# EUROPÄISCHE PATENTSCHRIFT

(11) **EP 3 900 923 B1**
(45) Veröffentlichungstag und Bekanntmachung des Hinweises auf die Patenterteilung: **01.11.2023**
(21) Anmeldenummer: 20170820.3
(22) Anmeldetag: 22.04.2020
(51) Int. Cl.: B30B 15/30, B30B 11/02, B30B 11/00

(54) **VERFAHREN ZUM AUTOMATISIERTEN HERSTELLEN VON INDIVIDUALISIERTEN TABLETTEN UND TABLETTENPRESSE ZUR AUTOMAITISIERTEN HERSTELLUNG VON INDIVIDUALISIERTEN TABLETTEN**
METHOD FOR THE AUTOMATIC PRODUCTION OF INDIVIDUALIZED TABLETS AND TABLET PRESS FOR THE AUTOMATED PRODUCTION OF INDIVIDUALIZED TABLETS
PROCÉDÉ DE FABRICATION AUTOMATISÉE DE COMPRIMÉS INDIVIDUALISÉS ET PRESSE À COMPRIMÉS DESTINÉE À LA FABRICATION AUTOMATISÉE DE COMPRIMÉS INDIVIDUALISÉS

(43) Veröffentlichungstag der Anmeldung: 27.10.2021
(73) Patentinhaber: PrivMed X AB, 184 92 Åkersberga (SE)
(72) Erfinder: KRAUSE, Ingo, 23683 Scharbeutz (DE); MANIGK, Rene, 73113 Ottenbach (DE)
(74) Vertreter: Seemann & Partner Patentanwälte mbB

(56) Entgegenhaltungen:
- EP-A2- 1 844 924
- WO-A1-2014/001805
- WO-A2-2007/022336
- CH-A5- 668 032
- CN-A- 107 718 675
- GB-A- 1 183 354

## Beschreibung

Die Erfindung betrifft ein Verfahren zum automatisierten Herstellen einer vorgebbaren Anzahl von individualisierten Tabletten mit einer vorgebbaren Menge eines ersten Wirkstoffs mittels einer Tablettenpresse.

Die Erfindung betrifft weiterhin eine Tablettenpresse zur automatisierten Herstellung einer vorgebbaren Anzahl von individualisierten Tabletten mit einer vorgebbaren Menge eines ersten Wirkstoffs.

Tabletten spielen heutzutage eine unverzichtbare Rolle in medizinischen und nicht medizinischen Bereichen, angefangen von der Verabreichung von Medikamenten bis hin zu Nahrungsergänzungsmitteln.

Zur Tablettenherstellung wird vielfach eine Matrize mit einer zentralen Öffnung eingesetzt, in die der Wirkstoff gefüllt wird, aus dem die Tablette hergestellt werden soll. Mittels eines Unterstempels und eines Oberstempels wird der Wirkstoff in der zentralen Öffnung zu einer Tablette gepresst.

Um den großen Bedarf an Tabletten zu decken, werden die Tabletten vielfach in standardisierten Größen mit einer festgelegten Menge an Wirkstoff produziert. Hierzu kommen beispielsweise Rundläuferpressen zum Einsatz, die bis zu hundert Matrizen und Stempelpaare umfassen, welche mittels einer Drehplatte rotiert und synchronisiert angetrieben werden. Solche Rundläuferpressen können bis zu 1,6 Millionen Tabletten pro Stunde herstellen.

CN 107 718 675 A zeigt eine Rundläuferpresse, die zur Herstellung von Tabletten vorgesehen ist, welche für Airbags von Autos verwendet wird. Eine obere Stempelgruppe und eine untere Stempelgruppe umfassen jeweils eine Vielzahl von Oberstempeln und Unterstempeln, die mittels mehrerer Pressräder in eine Öffnung einer Mittelschiene gedrückt werden. Eine in Umfangsrichtung versetzt angeordnete Befüllvorrichtung befüllt die Öffnung zuvor mit dem zu pressenden Pulver.

Gerade bei medizinischen Anwendungen sind die zu verabreichenden Wirkstoffmengen jedoch abhängig von den individuellen Patienten, der Wirkstoffaufnahmefähigkeit und dem derzeitigen Gesundheitszustand dieser Patienten. Besonders, aber nicht nur, Kinder oder ältere Menschen bedürfen einer speziell abgestimmten Dosieranpassung.

Da gängige medizinische Wirkstoffpräparate meist nur in einer Anzahl von Standarddosierungen verfügbar sind, müssen Arzt und Apotheker fast immer eine Überdosierung wählen, da eine Unterdosierung nicht den angestrebten Behandlungserfolg hätte. Überdosierungen belasten jedoch das Organsystem der Patienten unnötig und verursachen auf Dauer Schäden. Das Aufteilen von Tabletten in kleinere Stücke mittels eines Messers ist für Patienten unpraktikabel und nicht zielführend.

Zudem werden Tabletten vielfach in Tablettenverpackungen mit einer vorgegebenen Anzahl an Tabletten verkauft. Dies führt zu einer Verschwendung von Pharmarohstoffen und überschüssigen Tabletten, die vom Patienten nicht benötigt und weggeworfen werden.

Es ist die Aufgabe der vorliegenden Erfindung, die für Patienten individualisierte Dosierung und Einnahme von in Tablettenform verabreichten Wirkstoffen zu vereinfachen.

Diese Aufgabe wird gelöst durch ein Verfahren zum automatisierten Herstellen einer vorgebbaren Anzahl von individualisierten Tabletten mit einer vorgebbaren Menge eines ersten Wirkstoffs mittels einer, insbesondere kompakten, Tablettenpresse mit den folgenden Verfahrensschritten:
- Eingabe einer Sollanzahl, die die Anzahl herzustellender Tabletten angibt, und einer Sollmasse, die die Menge des ersten Wirkstoffs pro Tablette angibt, in eine Eingabevorrichtung,
- Übermitteln der Sollanzahl und der Sollmasse von der Eingabevorrichtung an ein Steuerungssystem der Tablettenpresse, wobei das Steuerungssystem eine erste Dosiervorrichtung, eine Wiegevorrichtung, einen vertikal auslenkbaren Oberstempel und einen vertikal auslenkbaren Unterstempel steuert,
- Abfüllen einer Menge des ersten Wirkstoffs, die der Sollmasse entspricht, aus einem ersten Materialbehälter mittels der ersten Dosiervorrichtung in eine zentrale Öffnung einer Matrize, wobei während des Abfüllens eine Druckfläche des Unterstempels die zentrale Öffnung verschließt und mittels der Wiegevorrichtung, auf die der Unterstempel und die Matrize während des Abfüllens abgesetzt werden, die in die zentrale Öffnung abgefüllte Menge des ersten Wirkstoffs gemessen wird,

- Pressen des ersten Wirkstoffs durch Auslenken einer Druckfläche des Oberstempels in die zentrale Öffnung,
- Auslösen einer gepressten Tablette aus der Matrize durch Auslenken der Druckfläche des Unterstempels in die zentrale Öffnung,
- Wiederholen der Verfahrensschritte des Abfüllens der Menge des ersten Wirkstoffs, des Pressens des ersten Wirkstoffs, und des Auslösens der gepressten Tablette, bis die Anzahl der gepressten Tabletten der Sollanzahl entspricht.

Insbesondere wird vor dem Verfahren ein Tablettenbehälter in einem Aufnahmeraum der Tablettenpresse angeordnet und nach dem Auslösen der gepressten Tablette diese Tablette mittels einer von dem Steuerungssystem gesteuerten Transfervorrichtung in den Tablettenbehälter transferiert.

Durch dieses Verfahren werden dem Patienten eine vorgebbare Sollanzahl individualisierte Tabletten mit einer vorgebbaren Sollmasse des ersten Wirkstoffs zur Verfügung gestellt. Die Sollmasse wird beispielsweise von einem Arzt oder einem Apotheker unter Berücksichtigung des Gesundheitszustands und der körperlichen Eigenschaften des Patienten, wie beispielsweise seinem Körpergewicht, festgelegt. Da die in der Tablette enthaltene Wirkstoffmenge exakt der Sollmasse entspricht, ist ein Zerteilen der Tablette überflüssig. Auf diese Weise werden vorteilhaft Überdosierungen vermieden und dem Patienten die Einnahme der Tabletten erleichtert.

Durch das Anordnen des Unterstempels und der Matrize auf der Wiegevorrichtung lässt sich die in die zentrale Öffnung eingefüllte Masse des ersten Wirkstoffes während des Abfüllens exakt und zuverlässig messen.

Die Sollanzahl wird beispielsweise ebenfalls von dem Arzt oder dem Apotheker festgelegt. Dabei wird bevorzugt berücksichtigt, wie schnell sich der Gesundheitszustand des Patienten verändert und wie häufig eine Anpassung der Dosierung erforderlich wird. Die Sollanzahl wird bevorzugt so gewählt, dass die hergestellten Tabletten für die Behandlung des Patienten bis zur nächsten Diagnose ausreichen, ohne dass überflüssige Tabletten hergestellt werden. Eine Verschwendung von Tabletten durch die Abgabe von standardisierten Tablettenverpackungen, die mehr als die benötigten Tabletten enthalten, wird vorteilhaft vermieden.

Vorteilhaft ist die Tablettenpresse kompakt. Dies wird insbesondere dadurch erreicht, dass die Tablettenpresse nur einen einzelnen Oberstempel und/oder einen einzelnen Unterstempel umfasst. Die Tablettenpresse befindet sich beispielsweise in einer Arztpraxis, einer Apotheke oder beim Patienten zu Hause. Bevorzugt werden die Tabletten direkt nach der Ermittlung der Sollmasse und der Sollanzahl in der Arztpraxis, in der Apotheke oder vom Patienten selbst bei sich zu Hause hergestellt. Es wird somit ein dezentrales Verfahren zur Herstellung von individualisierten Tabletten außerhalb von industriellen Fertigungsanlagen zur Verfügung gestellt. Da die Anzahl der von einem Patienten benötigen individualisierten Tabletten vergleichsweise klein ist, ist für ihre Herstellung keine hohe Fertigungsgeschwindigkeit notwendig. Stattdessen ist es vorteilhafterweise vorgesehen, dass eine Vielzahl von erfindungsgemäßen Tablettenpressen in einer Vielzahl von Arztpraxen, Apotheken oder Patientenwohnungen den Bedarf an individualisierten Tabletten der Patienten decken.

Der erste Materialbehälter ist insbesondere lösbar an einer ersten Dosieröffnung, insbesondere eines Pressraums, der Tablettenpresse fixiert. Dadurch lässt sich vorteilhaft der erste Materialbehälter schnell austauschen, beispielsweise wenn Tabletten mit einem anderen Wirkstoff hergestellt werden sollen. Die erste Dosiervorrichtung ist insbesondere ein Bestandteil des ersten Materialbehälters und bildet mit diesem eine Einheit. Der erste Wirkstoff ist entweder ein einzelner Wirkstoff oder eine Mischung von verschiedenen Wirkstoffen.

Der Oberstempel und/oder der Unterstempel sind insbesondere vertikal auslenkbar. Insbesondere entspricht der Außenumfang der Druckfläche des Oberstempels und/oder der Druckfläche des Unterstempels einem Innenumfang der zentralen Öffnung der Matrize. Insbesondere verschließt die Druckfläche des Unterstempels die zentrale Öffnung von unten, indem die Druckfläche des Unterstempels von unten in die zentrale Öffnung hineingeschoben wird. Dadurch entsteht eine Mulde, deren Wände von der zentralen Öffnung und deren Boden von der Druckfläche des Unterstempels gebildet werden. Insbesondere wird zum Pressen des ersten Wirkstoffs die Druckfläche des Oberstempels während des Pressens vertikal von oben in die zentrale Öffnung ausgelenkt. Zum Auslösen der gepressten Tablette wird die Druckfläche des Oberstempels zurück nach oben gefahren und die Druckfläche des Unterstempels nach oben ausgelenkt, bis sie insbesondere plan mit der Oberkante der Matrize ist.

Die Eingabevorrichtung umfasst Eingabemittel zur Eingabe der Sollmasse und der Sollanzahl und ist insbesondere ein berührungsempfindlicher Bildschirm. Insbesondere ist die Eingabevorrichtung zudem dazu eingerichtet, auf einer Datenanzeige die eingegebene Sollanzahl und/oder die eingegebene Sollmasse und/oder zusätzliche Informationen, insbesondere Patientendaten und/oder Information über den ersten Wirkstoff, anzuzeigen. Weiterhin umfasst die Eingabevorrichtung ein Eingabemittel, mit dem die Herstellung der Tabletten gestartet wird. Insbesondere umfasst die Eingabevorrichtung ein Zugangssicherungssystem. Beispielsweise ist die Eingabevorrichtung mittels eines Zugangscodes gesichert oder umfasst einen Fingerabdruckscanner. Gemäß einer Ausführungsform ist die Eingabevorrichtung ein fester Bestandteil der Tablettenpresse. Gemäß einer anderen Ausführungsform ist die Eingabevorrichtung ein externes Gerät, das die Sollanzahl und die Sollmasse an das Steuerungssystem übermittelt. Gemäß einer weiteren Ausführungsform ist die Eingabevorrichtung eine Schnittstelle der Tablettenpresse, über die die Sollanzahl und die Sollmasse von einem externen Gerät an das Steuerungssystem übermittelt werden.

Insbesondere sind die Sollanzahl und die Sollmasse in einem Kennzeichen, insbesondere einem Strichcode codiert, das insbesondere auf einem dem Patienten ausgestellten Rezept aufgedruckt ist. Vorzugsweise wird das Kennzeichen mittels einer Bildaufnahmevorrichtung der Eingabevorrichtung, insbesondere einer Kamera, eingelesen und an das Steuerungssystem übermittelt, wobei das Steuerungssystem aus dem Kennzeichen die Sollanzahl und die Sollmasse bestimmt.

Gemäß einer Ausführungsform ist der Pressraum lösbar in der Tablettenpresse fixiert. Insbesondere ist der Pressraum zusammen mit dem darin angeordneten Unterstempel und der darin angeordneten Matrize als wechselbarer Pressraumeinschub ausgestaltet.

Das Steuerungssystem ist insbesondere ein Computer, der dazu eingerichtet ist, die in die Eingabevorrichtung eingegebene Sollmasse und Sollanzahl auszulesen und die erste Dosiervorrichtung, die Wiegevorrichtung, den Oberstempel, den Unterstempel und die Transfervorrichtung anzusteuern.

Die Transfervorrichtung umfasst insbesondere eine Schubvorrichtung, mittels derer die hergestellte Tablette von der Druckfläche des Unterstempels heruntergeschoben wird. Weiterhin insbesondere umfasst die Transfervorrichtung einen Kanal, der oberhalb eines in dem Aufnahmeraum angeordneten Tablettenbehälters mündet. Durch den Kanal fallen die von der Druckfläche des Unterstempels heruntergeschobenen Tabletten in den Tablettenbehälter. Direkt nach dem Transferieren der gepressten Tablette wird die nächste Tablette hergestellt, bis die Sollanzahl erreicht ist.

Gemäß einer Ausführungsform berechnet das Steuerungssystem unter Berücksichtigung der Sollmasse, und gegebenenfalls einer Schüttdichte, des ersten Wirkstoffs ein Füllvolumen und damit einen Sollumfang, wählt aus wenigstens zwei in der Tablettenpresse angeordneten Matrizen mit unterschiedlichen Innenumfängen eine Sollmatrize aus, deren Innenumfang ihrer zentralen Öffnung dem Sollumfang entspricht, und steuert eine erste Positioniervorrichtung an, so dass die Sollmatrize über dem Unterstempel angeordnet wird, wobei insbesondere die erste Positioniervorrichtung eine Schienenvorrichtung ist, an der die wenigstens zwei in der Tablettenpresse angeordneten Matrizen angeordnet, insbesondere fixiert, sind.

Vorteilhaft ist auf diese Weise die Größe der gepressten Tabletten variierbar. Das Steuersystem bestimmt dafür automatisch anhand der Sollmasse, und gegebenenfalls der Schüttdichte, des ersten Wirkstoffs ein Füllvolumen und damit einen passenden Sollumfang, wählt eine passende Sollmatrize aus und ordnet diese über dem Unterstempel an. Ein manueller Austausch der Matrize ist somit überflüssig. Durch die Variabilität der Größe der gepressten Tabletten lassen sich individualisierte Tabletten mit stark unterschiedlichen Wirkstoffmengen herstellen.

Insbesondere sind auf der ersten Positioniervorrichtung wenigstens drei Matrizen mit unterschiedlichen Innenumfängen ihrer zentralen Öffnungen fixiert.

Weiterhin bevorzugt wird der Außenumfang der Druckfläche des Oberstempels zum Pressen des ersten Wirkstoffs an den Innenumfang der Matrize angepasst, indem eine Anzahl von vertikal teleskopierend ineinander angeordneten Oberstempelschäften des Oberstempels nach unten ausgelenkt werden, wobei der Außenumfang des äußeren ausgelenkten Oberstempelschafts dem Innenumfang der Matrize entspricht.

Durch das Anpassen des Außenumfangs der Druckfläche des Oberstempels an den Innenumfang der Matrize wird vorteilhaft ein Auswechseln des Oberstempels beim Einsatz von Matrizen mit unterschiedlichen Innenumfängen überflüssig. Das Anpassen des Außenumfangs der Druckfläche des Oberstempels erfolgt durch das Auslenken der Oberstempelschäfte. Die Druckfläche des Oberstempels entspricht der Gesamtheit der Druckflächen der ausgelenkten Oberstempelschäfte. Insbesondere sind die Druckflächen der Oberstempelschäfte so ausgebildet, dass die Druckfläche des Oberstempels stets kontinuierlich ist.

Insbesondere umfasst der Oberstempel für jede auf der ersten Positioniervorrichtung angeordnete Matrize einen zugeordneten Oberstempelschaft, dessen Querschnittsform der Querschnittsform der zentralen Öffnung einer der Matrizen entspricht. Insbesondere wird zum Pressen des ersten Wirkstoffs stets der der zentralen Öffnung zugeordnete Oberstempelschaft und alle weiter innen angeordneten Oberstempelschäfte ausgelenkt. Die Oberstempelschäfte sind insbesondere im Wesentlichen spaltfrei ineinander angeordnet. Auf diese Weise ist die Druckfläche des Oberstempels stets nutfrei, so dass Tabletten mit glatter Oberfläche hergestellt werden.

Vorzugsweise wird der Außenumfang der Druckfläche des Unterstempels vor dem Abfüllen der Menge des ersten Wirkstoffs an den Innenumfang der Matrize angepasst, indem eine Anzahl von vertikal teleskopierend ineinander angeordneten Unterstempelschäften des Unterstempels nach oben ausgelenkt werden, wobei der Außenumfang des äußeren ausgelenkten Unterstempelschafts dem Innenumfang der Matrize entspricht.

Durch das Anpassen des Außenumfangs der Druckfläche des Unterstempels an den Innenumfang der Matrizen wird vorteilhaft auch ein Auswechseln des Unterstempels beim Einsatz von Matrizen mit unterschiedlichen Innenumfängen überflüssig. Insbesondere wird der Außenumfang der Druckfläche des Unterstempels an den Innenumfang der Matrizen angepasst, bevor die Druckfläche des Unterstempels von unten in die zentrale Öffnung ausgelenkt wird, um diese von unten zu verschließen.

Die Unterstempelschäfte sind bevorzugt spiegelbildlich zu den Oberstempelschäften gefertigt, so dass die im Hinblick auf die Oberstempelschäfte beschriebenen Vorteile und Eigenschaften auch auf die Unterstempelschäfte zutreffen.

Gemäß einer Ausführungsform bilden der Unterstempel und die Matrize eine Einheit, wobei Unterstempel und Matrize insbesondere in vertikaler Richtung zueinander bewegbar sind. Insbesondere sind der Unterstempel und die Matrize mittels einer Feder gekoppelt. Die Feder zieht den Unterstempel nach dem Auslenken des Unterstempels durch ihre Federkraft wieder zurück in die Ausgangslage.

Die Masse des in die zentrale Öffnung abgefüllten ersten Wirkstoffes wird vorzugsweise gemessen, indem vor dem Abfüllen die Matrize und der Unterstempel auf der Wiegevorrichtung abgesetzt werden, insbesondere mittels wenigstens einer Hubeinrichtung, wobei die Wiegevorrichtung während des Abfüllens die auf sie wirkende Gesamtmasse misst, die die Masse der Matrize, die Masse des Unterstempels sowie die Masse des in die zentrale Öffnung gefüllten ersten Wirkstoffs umfasst, wobei die Matrize und der Unterstempel nach dem Abfüllen und vor dem Pressen des ersten Wirkstoffes angehoben werden, insbesondere mittels der wenigstens einen Hubeinrichtung.

Nach dem Absetzten der Matrize und des Unterstempels und vor dem Abfüllen wird insbesondere die Gesamtmasse gemessen, um einen Messwert für den nicht befüllten Zustand zu erhalten. Dieser Messwert wird während des Abfüllens von der Gesamtmasse subtrahiert, um die Masse des in die zentrale Öffnung gefüllten ersten Wirkstoffs zu berechnen. Das Abfüllen wird beendet, wenn die Masse des in die zentrale Öffnung gefüllten ersten Wirkstoffs der Sollmasse entspricht. Zur Steuerung des Abfüllvorgangs werden die von der Wiegevorrichtung aufgenommenen Messdaten der Gesamtmasse an das Steuerungssystem übermittelt. Dieses berechnet aus den Messdaten der Gesamtmasse die derzeitige Masse des in die zentrale Öffnung gefüllten ersten Wirkstoffs. Ist die Sollmasse erreicht, steuert sie die Dosiervorrichtung des ersten Materialbehälters an, um das Abfüllen zu beenden. Insbesondere koppelt die Steuervorrichtung die Wiegevorrichtung mit der ersten Dosiervorrichtung mittels eines Algorithmus.

Nach dem Anheben liegen die Matrize und der Unterstempel nicht mehr auf der Wiegevorrichtung auf, so dass die Wiegevorrichtung während des Pressens nicht beschädigt wird. Das Anheben und Absetzen wird insbesondere mittels wenigstens einer Hubeinrichtung durchgeführt. Die Hubeinrichtung ist insbesondere dazu eingerichtet, die Matrize und den Unterstempel in vertikaler Richtung zu bewegen. Insbesondere liegen der Unterstempel und/oder die wenigstens eine Matrize auf einer Plattform auf, die von der wenigstens einen Hubeinrichtung in vertikaler Richtung bewegbar ist. Nachdem der Unterstempel und die Matrize auf der Wiegevorrichtung abgesetzt wurden, wird insbesondere die Plattform soweit nach unten gefahren, dass der Unterstempel und die Matrize während des Abfüllens ausschließlich auf der Wiegevorrichtung und nicht mehr auf der Plattform aufliegen.

Vorzugsweise wird der auf den Unterstempel und/oder den Oberstempel wirkende Druck an der wenigstens einen Hubeinrichtung, insbesondere mittels einer Druckmesseinrichtung, gemessen.

Auf diese Weise kann der wirkende Druck während des Pressvorgangs gemessen werden. Die Druckmesseinrichtung ist insbesondere ein Kraftaufnehmer im Sinne einer Druckmessdose. Vorteilhaft lässt sich dadurch der Druck während der Herstellung der Tabletten überwachen, eine Tablettenhärte und/oder Bruchfestigkeit der Tabletten steuern und eine Beschädigung der Stempel und/oder Matrizen verhindern.

Bevorzugt wird vor dem Abfüllen der Menge des ersten Wirkstoffs die erste Dosiervorrichtung mittels einer zweiten Positioniervorrichtung, insbesondere einer in horizontaler Richtung bewegbaren Schienenvorrichtung, vertikal über der zentralen Öffnung angeordnet, wobei nach dem Abfüllen der Menge des ersten Wirkstoffs und vor dem Pressen des ersten Wirkstoffs der Oberstempel mittels der zweiten Positioniervorrichtung vertikal über der zentralen Öffnung angeordnet wird, wobei die erste Dosiervorrichtung insbesondere auch in vertikaler Richtung bewegbar an der zweiten Positioniervorrichtung angeordnet, insbesondere fixiert, ist.

Durch die zweite Positioniervorrichtung wird vorteilhaft das Abfüllen des ersten Wirkstoffs erleichtert. Durch Anordnen der ersten Dosiervorrichtung über der zentralen Öffnung kann das Abfüllen des ersten Wirkstoffs direkt und verlustfrei von oben erfolgen. Nach dem Abfüllen des ersten Wirkstoffs wird die erste Dosiervorrichtung zur Seite bewegt, so dass der Oberstempel über der zentralen Öffnung angeordnet wird.

Insbesondere wird die erste Dosiervorrichtung während des Abfüllens des ersten Wirkstoffs mittels der zweiten Positioniervorrichtung in horizontaler Richtung bewegt. Insbesondere wird die erste Dosiervorrichtung während des Abfüllens mittels der zweiten Positioniervorrichtung in horizontaler Richtung wiederholt von links nach rechts in der Matrize bewegt. Dies ist gerade beim Befüllen von großen und/ oder ovalen Matrizen vorteilhaft um den Wirkstoff gleichmäßig zu verteilen.

Insbesondere wird eine zweite Dosiervorrichtung mittels der zweiten Positioniervorrichtung in horizontaler Richtung bewegt. Auf diese Weise ist es möglich, eine Tablette mit zwei nebeneinanderliegenden Schichten herzustellen. Dazu wird die erste Dosiervorrichtung über eine erste Ecke der Matrize und die zweite Dosiervorrichtung über eine zweite Ecke der Matrize bewegt, die erste Ecke mittels der ersten Dosiervorrichtung mit dem ersten Wirkstoff und die zweite Ecke mittels der zweiten Dosiervorrichtung mit einem zweiten Wirkstoff befüllt und anschließend eine Zweischichttablette mit nebeneinanderliegenden Schichten gepresst.

Eine Schienenvorrichtung stellt eine zuverlässige und kompakte Positioniervorrichtung dar, durch die sich die erste Dosiervorrichtung und der Oberstempel in horizontaler Richtung bewegen lassen. Indem die erste Dosiervorrichtung zusätzlich in vertikaler Richtung bewegbar ist, lässt sich die erste Dosiervorrichtung nach der Positionierung der ersten Dosiervorrichtung über der zentralen Öffnung soweit absenken, dass die Unterkante der ersten Positioniervorrichtung unter der Oberkante der wenigstens einen Matrize angeordnet ist. Mit anderen Worten wird die erste Dosiervorrichtung in die zentrale Öffnung hinein abgesenkt. Auf diese Weise wird ein Verschütten des Wirkstoffes während des Abfüllens verhindert, so dass die gesamte Menge des Wirkstoffes in die zentrale Öffnung gelangt.

Weiterhin wird die Aufgabe gelöst durch ein Verfahren zum Herstellen einer vorgebbaren Anzahl individualisierter Zweischichttabletten mit einer vorgebbaren Menge des ersten Wirkstoffs und einer vorgebbaren Menge eines zweiten Wirkstoffs, wobei die Verfahrensschritte des zuvor beschriebenen Verfahrens mit folgenden zusätzlichen Verfahrensschritten ausgeführt werden:
- Eingeben einer Zweitschichtsollmasse in die Eingabevorrichtung und Übermitteln der Zweitschichtsollmasse an das Steuerungssystem, wobei die Zweischichtsollmasse die Menge des zweiten Wirkstoffs pro Zweischichttablette angibt,
- Positionieren einer zweiten Dosiervorrichtung eines zweiten Materialbehälters mittels der zweiten Positioniervorrichtung über der zentralen Öffnung,
- Abfüllen einer Menge eines zweiten Wirkstoffs, die der Zweitschichtsollmasse entspricht, mittels der zweiten Dosiervorrichtung in die zentrale Öffnung,

- Positionieren des Oberstempels mittels der zweiten Positioniervorrichtung über der zentralen Öffnung,
- Pressen des zweiten Wirkstoffs durch vertikales Auslenken der Druckfläche des Oberstempels in die zentrale Öffnung,
wobei bis auf das Eingeben der Zweitschichtsollmasse und das Übermitteln der Zweitschichtsollmasse alle zusätzlichen Verfahrensschritte nach dem Pressen des ersten Wirkstoffs und vor dem Ausgeben der gepressten Tablette ausgeführt werden.

Vor der Durchführung dieses Verfahrens wird der zweite Materialbehälter, der mit dem zweiten Wirkstoff befüllt ist, derart an der Tablettenpresse fixiert, dass er mittels der zweiten Positioniervorrichtung in horizontaler Richtung bewegbar ist. Insbesondere umfassen der erste Materialbehälter und/oder der zweite Materialbehälter einen RFID Chip. Auf diese Weise kann die Tablettenpresse die Materialbehälter eindeutig identifizieren, egal an welcher Position die jeweiligen Materialbehälter in der Tablettenpresse eingebaut sind. Das Abmessen der Zweitschichtsollmasse wird ebenso durchgeführt wie das Abmessen der Sollmasse. Es wird also vor dem Abfüllen des zweiten Wirkstoffs die Matrize und der Unterstempel auf der Wiegevorrichtung abgesetzt und anschließend die Gesamtmasse gemessen. Das Pressen des ersten Wirkstoffs erfolgt insbesondere mit einem kleineren Druck als das Pressen des zweiten Wirkstoffs. Auf diese Weise wird durch das Pressen des ersten Wirkstoffs eine Kavität für den zweiten Wirkstoff geschaffen und verhindert, dass die zwei Tablettenhemisphären nach dem Pressen des zweiten Wirkstoffs auseinanderfallen.

Durch dieses Verfahren lassen sich vorteilhaft individualisierte Zweischichttabletten herstellen, die an die Patientenbedürfnisse angepasst sind. Nach dem Herstellen einer ersten Schicht durch Abfüllen und Pressen des ersten Wirkstoffs wird mittels der zweiten Positioniervorrichtung die zweite Dosiervorrichtung über der zentralen Öffnung angeordnet und die zentrale Öffnung mit der Zweitschichtsollmasse des zweiten Wirkstoffs aufgefüllt. Anschließend wird mittels des Oberstempels der zweite Wirkstoff zu einer zweiten Schicht über der ersten Schicht gepresst. Anschließend wird die hergestellte Zweischichttablette auf die zuvor beschriebene Weise aus der Matrize ausgelöst und in den Tablettenbehälter transferiert.

Die Aufgabe wird außerdem gelöst durch eine Tablettenpresse zur automatisierten Herstellung einer vorgebbaren Anzahl von individualisierten Tabletten mit einer vorgebbaren Menge eines ersten Wirkstoffs, umfassend einen Pressraum, einen mit dem ersten Wirkstoff befüllbaren ersten Materialbehälter, eine erste Dosiervorrichtung, ein Steuerungssystem und eine Eingabevorrichtung, wobei der Pressraum einen auslenkbaren Oberstempel, einen auslenkbaren Unterstempel, wenigstens eine Matrize und eine unter der wenigstens einen Matrize angeordnete Wiegevorrichtung aufweist, mittels der eine Masse eines in eine zentrale Öffnung der Matrize gefüllten Materials durch Absetzen der Matrize und des Unterstempels auf der Wiegevorrichtung messbar ist, wobei die Eingabevorrichtung dazu eingerichtet ist, eine Sollanzahl und eine Sollmasse der herzustellenden individualisierten Tabletten an das Steuerungssystem zu übermitteln, und wobei das Steuerungssystem dazu eingerichtet ist, Messdaten der Wiegevorrichtung auszulesen und die erste Dosiervorrichtung, den Oberstempel und den Unterstempel derart anzusteuern, dass eine Menge des ersten Wirkstoffs, die der Sollmasse entspricht, in die zentrale Öffnung der Matrize gefüllt und mittels des Oberstempels und des Unterstempels zu einer Tablette gepresst wird, und diesen Vorgang so oft zu wiederholen, bis die Sollanzahl an Tabletten hergestellt wurde.

Die Tablettenpresse verkörpert die gleichen Vorteile, Merkmale und Eigenschaften wie das zuvor beschriebene Verfahren zum automatisierten Herstellen einer vorgebbaren Anzahl von individualisierten Tabletten mit einer vorgebbaren Menge eines ersten Wirkstoffs mittels einer Tablettenpresse.

Vorteilhaft stellt die erfindungsgemäße Tablettenpresse eine dezentrale Vorrichtung zur Herstellung von individualisierten Tabletten dar. Die individualisierten Tabletten werden direkt vor Ort, also in einer Praxis, einer Apotheke oder einer Pharmaproduktion von fachkundigen Ärzten oder Apothekern oder von entsprechenden unterwiesenen Patienten hergestellt. Durch die individualisierten Tabletten werden Fehldosierungen/Überdosierungen vermieden und dem Patienten die Einnahme der Tabletten erleichtert, da diese nicht mehr zerteilt werden müssen.

Der Oberstempel und/oder der Unterstempel sind insbesondere vertikal auslenkbar. Gemäß einer Ausführungsform ist der Unterstempel ortsfest im Pressraum angeordnet. Die Position des Unterstempels im Pressraum ändert sich somit gemäß dieser Ausführungsform in horizontaler Richtung nicht, der Unterstempel wird lediglich in vertikaler Richtung ausgelenkt und angehoben beziehungsweise abgesenkt. Dadurch lässt sich die auf den Unterstempel wirkende Presskraft gut messen.

Die gepresste Tablette wird durch Auslenken des Unterstempels in die zentrale Öffnung ausgelöst. Die Tablettenpresse umfasst insbesondere einen Aufnahmeraum zur Aufnahme eines Tablettenbehälters. Die erste Dosiervorrichtung ist insbesondere als Teil des ersten Materialbehälters oder separat zu diesem ausgebildet. Die Eingabevorrichtung umfasst insbesondere Eingabemittel zur Eingabe der Sollanzahl, die die Anzahl herzustellender individualisierter Tabletten angibt.

Die Tablettenpresse umfasst gemäß einer Ausführungsform wenigstens zwei Matrizen, deren zentrale Öffnungen unterschiedliche Innenumfänge aufweisen, wobei das Steuerungssystem dazu eingerichtet ist, unter Berücksichtigung der Sollmasse, und gegebenenfalls einer Schüttdichte, des ersten Wirkstoffs ein Füllvolumen und damit einen Sollumfang zu berechnen, aus den wenigstens zwei Matrizen eine Sollmatrize auszuwählen, deren Innenumfang ihrer zentralen Öffnung dem Sollumfang entspricht, und eine erste Positioniervorrichtung so anzusteuern, dass die Sollmatrize über dem Unterstempel angeordnet wird, wobei insbesondere die erste Positioniervorrichtung eine zwischen dem Oberstempel und dem Unterstempel angeordnete Schienenvorrichtung ist, an der die wenigstens zwei Matrizen angeordnet, insbesondere fixiert, sind.

Vorzugsweise umfasst der Oberstempel wenigstens zwei vertikal teleskopierend ineinander angeordnete Oberstempelschäfte, mittels derer ein Außenumfang einer Druckfläche des Oberstempels durch individuelle Auslenkung der Oberstempelschäfte an den Innenumfang der zentralen Öffnung der wenigstens einen Matrize anpassbar ist.

Weiterhin vorzugsweise umfasst der Unterstempel wenigstens zwei vertikal teleskopierend ineinander angeordnete Unterstempelschäfte, mittels derer ein Außenumfang einer Druckfläche des Unterstempels durch individuelle Auslenkung der Unterstempelschäfte an den Innenumfang der zentralen Öffnung der wenigstens einen Matrize anpassbar ist.

Bevorzugt umfasst der Pressraum eine erste Schub- und Zugvorrichtung, mittels derer wenigstens ein oberer Arretierbolzen in eine horizontale Bohrung in Seitenwände der Oberstempelschäfte einschiebbar ist, um eine festlegbare Anzahl der Oberstempelschäfte vertikal zu fixieren, wobei insbesondere der Pressraum eine zweite Schub-und Zugvorrichtung umfasst, mittels derer wenigstens ein unterer Arretierbolzen in eine horizontale Bohrung in Seitenwände der Unterstempelschäfte einschiebbar ist, um eine festlegbare Anzahl der Unterstempelschäfte vertikal zu fixieren.

Mittels der Arretierbolzen wird erreicht, dass beim Auslenken des Oberstempels und des Unterstempels nur die gewünschten Stempelschäfte ausgelenkt werden.

Der Oberstempel wird insbesondere über einen oberen Kolben ausgelenkt, der von oben auf den Oberstempel drückt. Der Unterstempel wird insbesondere entsprechend über einen unteren Kolben ausgelenkt, der von unten auf den Unterstempel drückt. Insbesondere sind der obere Kolben ausschließlich mit dem innersten Oberstempelschaft und der untere Kolben ausschließlich mit dem innersten Unterstempelschaft verbunden. Sollen zusätzlich weitere Oberstempelschäfte und/oder Unterstempelschäfte ausgelenkt werden, werden diese weiteren Oberstempelschäfte und/oder Unterstempelschäfte mittels eines Koppelbolzens mit dem inneren Oberstempelschaft und/oder Unterstempelschaft gekoppelt. Der Koppelbolzen wird insbesondere von einer stabförmigen Schub- und Zugvorrichtung angetrieben. Die Schub- und Zugvorrichtung ist insbesondere in einer vertikal laufenden Nut innerhalb eines Stempelträgers geführt, so dass sie die vertikale Bewegung der ausgelenkten Oberstempelschäfte und/oder Unterstempelschäfte mitvollziehen kann.

Bevorzugt umfasst die Tablettenpresse wenigstens eine Hubeinrichtung, die dazu eingerichtet ist, die wenigstens eine Matrize und den Unterstempel anzuheben und abzusenken, insbesondere in vertikaler Richtung.

Insbesondere werden die Matrize und der Unterstempel vor dem Abfüllen des ersten Wirkstoffs beziehungsweise des zweiten Wirkstoffs mittels der wenigstens einen Hubeinrichtung auf der Wiegevorrichtung abgesetzt und nach dem Abfüllen des ersten Wirkstoffs beziehungsweise des zweiten Wirkstoffs wieder angehoben.

Weiterhin vorzugsweise ist eine zweite Positioniervorrichtung umfasst, mittels derer wechselweise der Oberstempel und die erste Dosiervorrichtung, und insbesondere eine zweite Dosiervorrichtung eines zweiten Materialbehälters, vertikal über der zentralen Öffnung der über dem Unterstempel angeordneten Matrize anordbar sind.

Vorzugsweise ist die zweite Positioniervorrichtung eine Schienenvorrichtung ist, an der die erste Dosiervorrichtung und der Oberstempel angeordnet, insbesondere fixiert, und in horizontaler Richtung bewegbar sind, wobei die erste Dosiervorrichtung insbesondere auch in vertikaler Richtung bewegbar an der Schienenvorrichtung angeordnet, insbesondere fixiert, ist.

Gemäß einer bevorzugten Ausführungsform ist der Pressraum staubdicht, wobei insbesondere der Aufnahmeraum staubdicht verschließbar ist.

Vorteilhaft wird dadurch eine Kontamination des Aufnahmeraums und/oder eines in dem Aufnahmeraum angeordneten Tablettenbehälters und/oder des Bereichs außerhalb der Tablettenpresse vermieden.

Insbesondere bilden der Unterstempel und die Matrize eine Einheit. Insbesondere sind der Unterstempel und die Matrize mittels einer Feder in vertikaler Richtung miteinander gekoppelt.

Bevorzugt weist der Unterstempel an seiner Unterseite eine Ausnehmung auf, die formschlüssig zu einer Erhebung an der Oberseite der Wiegevorrichtung ausgebildet ist.

Auf diese Weise wird erreicht, dass der Unterstempel und die Matrize während des Abfüllens sicher auf der Wiegevorrichtung aufliegen und die Masse des abgefüllten Wirkstoffes exakt bestimmt wird.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1: eine schematisch vereinfachte Darstellung einer Tablettenpresse zur automatisierten Herstellung einer vorgebbaren Anzahl von individualisierten Tabletten mit einer vorgebbaren Menge eines ersten Wirkstoffs,
- Fig. 2: eine schematisch vereinfachte Darstellung einer Eingabevorrichtung der Tablettenpresse aus Fig. 1,
- Fig. 3: eine schematisch vereinfachte Querschnittsansicht eines Pressraums und eines Materialbehälters einer Tablettenpresse während des Abfüllens eines Wirkstoffs,
- Fig. 4: eine schematisch vereinfachte Querschnittsansicht eines Pressraums und eines Materialbehälters einer Tablettenpresse während des Pressens eines Wirkstoffs,
- Fig. 4b: eine schematisch vereinfachte Querschnittsansicht eines Unterstempels und einer Matrize, die mittels einer Feder gekoppelt sind, sowie einer Wiegevorrichtung,
- Fig. 4c: eine schematisch vereinfachte Querschnittsansicht des Unterstempels der Matrize, und der Wiegevorrichtung aus Fig. 4b während des Abfüllens,
- Fig. 5: eine schematisch vereinfachte Draufsicht auf eine erste Positioniervorrichtung,
- Fig. 6: eine schematisch vereinfachte Draufsicht auf eine zweite Positioniervorrichtung,
- Fig. 7a-d: schematisch vereinfachte Darstellungen eines Oberstempels mit teleskopierend ineinander angeordneten Oberstempelschäften und eines Unterstempels mit teleskopierend ineinander angeordneten Unterstempelschäften,
- Fig. 8a-c: schematisch vereinfachte Querschnittsansichten eines Oberstempels, eines Unterstempels und mehrerer Matrizen während des Pressvorgangs von Tabletten mit unterschiedlichen Wirkstoffmengen,
- Fig. 9a-c: schematisch vereinfachte Draufsichten auf einen Oberstempel während des Pressvorgangs von Tabletten mit unterschiedlichen Wirkstoffmengen.

In den Zeichnungen sind jeweils gleiche oder gleichartige Elemente und/oder Teile mit denselben Bezugsziffern versehen, so dass von einer erneuten Vorstellung jeweils abgesehen wird.

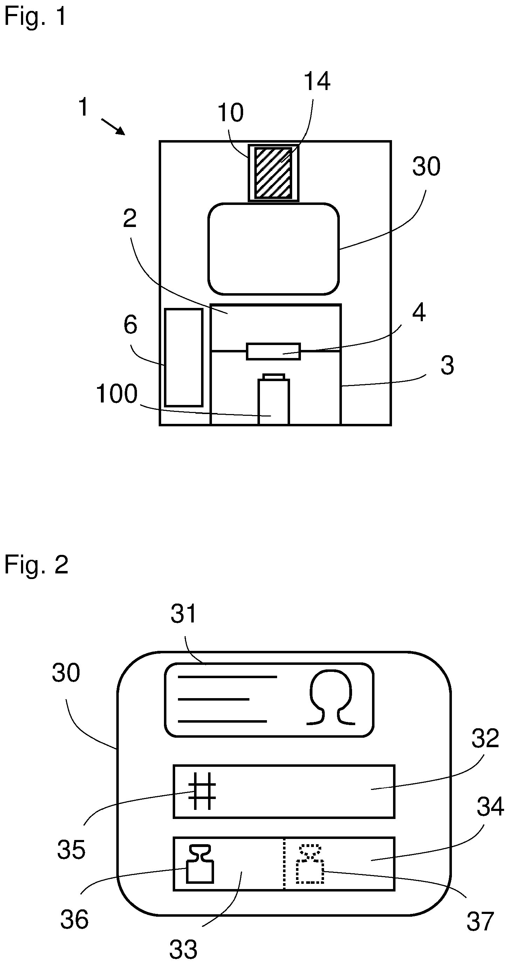

In Fig. 1 ist schematisch vereinfacht eine beispielhafte Ausführungsform einer erfindungsgemäßen Tablettenpresse 1 gezeigt. Die Tablettenpresse 1 umfasst einen Pressraum 2, einen ersten Materialbehälter 10, der mit einem ersten Wirkstoff 14 gefüllt ist, einen Aufnahmeraum 3, in dem ein Tablettenbehälter 100 anordbar ist, eine Transfervorrichtung 4 zum Transport der hergestellten Tabletten aus dem Pressraum 2 in den Tablettenbehälter 100, ein Steuerungssystem 6 zur Steuerung der Tablettenpresse 1 und eine Eingabevorrichtung 30 zur Bedienung der Tablettenpresse 1. Der erste Materialbehälter 10 ist in der in Fig. 1 gezeigten Ausführungsform der Tablettenpresse 1 in einem Gehäuse der Tablettenpresse 1 angeordnet und dort lösbar fixiert. Beispielsweise durch eine nicht dargestellte Klappe lässt sich der erste Materialbehälter 10 aus dem Gehäuse entfernen, um ihn auszutauschen oder wieder aufzufüllen. Insbesondere umfasst die Eingabevorrichtung 30 ein Zugangssicherungssystem. Beispielsweise ist die Eingabevorrichtung 30 mittels eines Zugangscodes gesichert oder umfasst einen Fingerabdruckscanner.

Gemäß einer Ausführungsform ist der Pressraum 2 als wechselbarerer Pressraumeinschub ausgestaltet. Dazu ist der Pressraum 2 lösbar in der Tabelettenpresse 1 fixiert. Zum Austauschen lässt sich der Pressraumeinschub aus der Tablettenpresse herausziehen und durch einen anderen Pressraumeinschub ersetzen.

Das Steuerungssystem 6 ist beispielsweise ein Computer, der dazu eingerichtet ist, die Tablettenpresse 1 so zu steuern, dass automatisiert eine Sollanzahl von individualisierten Tabletten mit einer Sollmasse des ersten Wirkstoffs 14 hergestellt wird. Der Aufnahmeraum 3 lässt sich gemäß einer bevorzugten Ausführungsform verschließen, so dass während der Herstellung der Tabletten kein Staub aus der Tablettenpresse 1 austritt und keine Verunreinigung in die Tablettenpresse 1 gelangen.

Fig. 2 zeigt schematisch vereinfacht eine Detailansicht der Eingabevorrichtung 30, bei der es sich in der gezeigten Ausführungsform um einen berührungsempfindlichen Bildschirm (englisch: "Touchscreen") an der Tablettenpresse 1 handelt. Die Eingabevorrichtung 30 umfasst eine Datenanzeige 31, beispielsweise ein Bereich des Bildschirms, auf der Informationen über den ersten Wirkstoff 14 und/oder einen Patienten angezeigt werden. Weiterhin umfasst die Eingabevorrichtung 30 mehrere Eingabemittel 32, 33, 34, bei denen es sich beispielsweise um weitere Bereiche des Bildschirms handelt. Mittels der Eingabemittel 32, 33, 34 lässt sich die Sollanzahl 35, die Sollmasse 36 und gegebenenfalls eine Zweitschichtsollmasse 37 eingeben. Die eingegebenen Werte werden zur Kontrolle angezeigt. Zudem ist ein nicht dargestelltes weiteres Eingabemittel vorgesehen, mit dem sich die Herstellung der Tabletten starten lässt. Gemäß anderer Ausführungsformen ist Eingabevorrichtung 30 ein externes Gerät oder eine Schnittstelle zur Kommunikation mit einem externen Gerät. In diesem Fall werden die Sollanzahl 35, die Sollmasse 36 und gegebenenfalls die Zweitschichtsollmasse 37 an dem externen Gerät eingeben und an das Steuerungssystem 6 übermittelt.

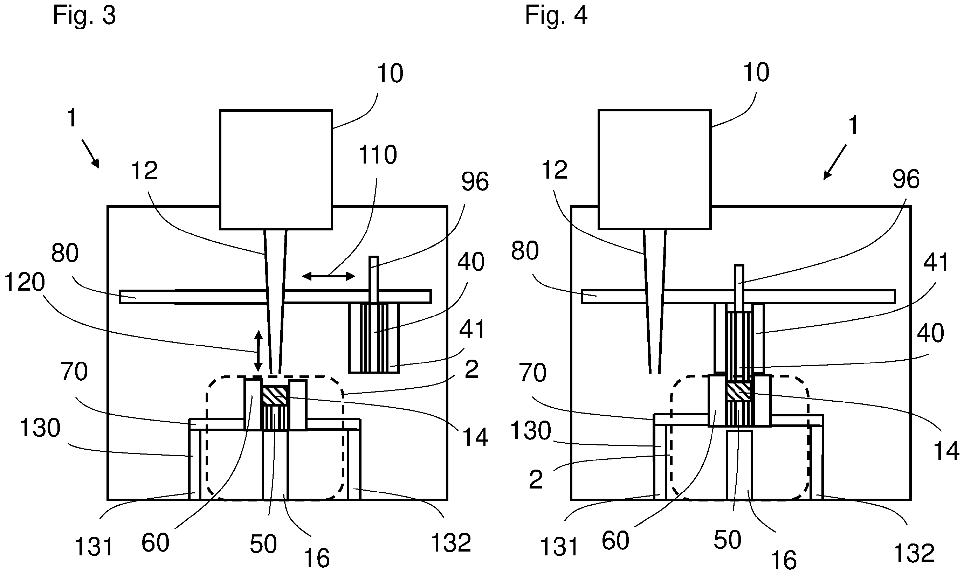

In Fig. 3 ist eine schematisch vereinfachte Querschnittsansicht einer Ausführungsform der Tablettenpresse 1 mit dem Pressraum 2 und dem ersten Materialbehälter 10 gezeigt. Der erste Materialbehälter 10 umfasst eine erste Dosiervorrichtung 12 zum Abfüllen des ersten Wirkstoffs 14 in die zentrale Öffnung einer Matrize 60. Gemäß einer anderen Ausführungsform ist der erste Materialbehälter 10 mit der ersten Dosiervorrichtung 12 verbunden. Die erste Dosiervorrichtung 12 ist beispielsweise ein verschließbarer Trichter.

Ein Unterstempel 50 ist derart im Pressraum 2 angeordnet, dass er von unten in die Matrize 60 hineinragt. Der Unterstempel 50 ist in vertikaler Richtung auslenkbar. Vor dem Abfüllen des ersten Wirkstoffs 14 in die Matrize 60 wird der Unterstempel 50 von unten in die Matrize 60 ausgelenkt, so dass eine Mulde zur Aufnahme des ersten Wirkstoffs 14 geschaffen wird. Alternativ ist der Unterstempel 50 dauerhaft in der Matrize 60 angeordnet. In beiden Fällen lässt sich der Unterstempel 50 in der zentralen Öffnung der Matrize in vertikaler Richtung 120 bewegen.

Die Matrize 60 und/oder der Unterstempel 50 sind auf einer Plattform 70 angeordnet. Gemäß einer Ausführungsform ist die Plattform 70 eine erste Positioniervorrichtung. Auf dieser ersten Positioniervorrichtung sind weitere, in Fig. 3 nicht dargestellte, Matrizen fixiert, welche sich durch die erste Positioniervorrichtung in eine Position oberhalb des Unterstempels 50 bewegen lassen. Gemäß einer alternativen Ausführungsform sind auf der Plattform 70 nur eine Matrize 60 und ein Unterstempel 50 fixiert.

Die Plattform 70 und damit auch die Matrize 60 und der Unterstempel 50 sind mittels einer Hubeinrichtung 130, die eine erste Spindel 131 und eine zweite Spindel 132 umfasst, in vertikaler Richtung 120 anhebbar und absenkbar. Über eine nicht gezeigte Druckmesseinrichtung an der Hubeinrichtung 130 und/oder über die Stromaufnahme der Hubeinrichtung 130 lässt sich zudem der während des Pressvorgangs auf den Unterstempel 50 wirkende Druck bestimmen. Unter der Matrize 60 und dem Unterstempel 50 ist eine Wiegevorrichtung16 angeordnet.

In der Tablettenpresse 1 ist weiterhin ein Oberstempel 40 angeordnet, der durch einen Oberstempelträger 41 umfasst und abgestützt wird. Mittels eines oberen Kolbens 96 lässt sich der Oberstempel 40 in vertikaler Richtung auslenken.

Die erste Dosiervorrichtung 12 und der Oberstempel 40 sind an einer zweiten Positioniervorrichtung 80 fixiert, bei der es sich in der gezeigten Ausführungsform um eine Schienenvorrichtung handelt.

Zum Befüllen der Matrize 60 mit dem ersten Wirkstoff 14 wird die zweite Positioniervorrichtung 80 von dem Steuerungssystem 6 angesteuert, so dass die erste Dosiervorrichtung 12 über dem Unterstempel 50 und der Matrize 60 angeordnet wird, wie in Fig. 3 gezeigt. Dies geschieht natürlich nur, sofern die erste Dosiervorrichtung 12 noch nicht über dem Unterstempel 50 angeordnet ist. Zudem werden die Matrize 60 und der Unterstempel 50 auf der Wiegevorrichtung 16 abgesetzt, so dass mittels der Wiegevorrichtung 16 das Gesamtgewicht der Matrize 60, des Unterstempels 50 sowie des in die Matrize gefüllten ersten Wirkstoffs 14 gemessen wird. Um ein Verschütten des ersten Wirkstoffes 14 während des Abfüllens zu verhindern, wir die erste Dosiervorrichtung 12 abgesenkt, so dass sich das untere Ende der ersten Dosiervorrichtung 12 unterhalb der Oberkante der Matrize 60 befindet. Anschließend wird die zentrale Öffnung der Matrize 60 mittels der ersten Dosiervorrichtung 12 mit dem ersten Wirkstoff 14 befüllt, bis die Sollmasse 36 erreicht ist. Dies wird mit der Wiegevorrichtung 16 überwacht.

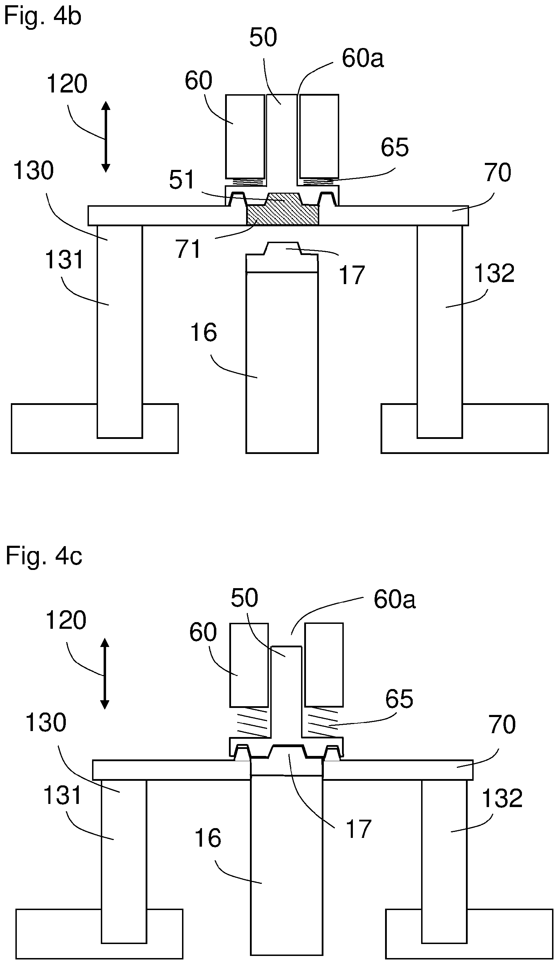

Nach dem Befüllen der Matrize 60 wird der erste Wirkstoff 14 gepresst. Dies ist in Fig. 4 gezeigt. Dazu wird die Matrize 60 und der Unterstempel 60 mittels der Hubeinrichtung 130 von der Wiegevorrichtung 16 angehoben, die Dosiervorrichtung 12 vertikal nach oben bewegt und die zweite Positioniervorrichtung 80 von dem Steuerungssystem 6 angesteuert, so dass der Oberstempel 40 über dem Unterstempel 50 und der Matrize 60 angeordnet wird. Anschließend wird der Oberstempel 40 mittels des oberen Kolbens 96 von oben in die zentrale Öffnung der Matrize 60 ausgelenkt. Auf diese Weise wird der erste Wirkstoff 14 zwischen den Druckflächen des Unterstempels 50 und des Oberstempels 40 zu einer Tablette gepresst.

Nach dem Pressen der Tablette bewegen sich sowohl der Oberstempel 40 als auch der Unterstempel 50 nach oben, um die Tablette aus der Matrize 60 zu lösen. Anschließend wird die Tablette beispielsweise mittels einer nicht dargestellten Schubvorrichtung der Transfervorrichtung 4 von dem Unterstempel 50 heruntergeschoben, so dass sie durch einen Kanal der Transfervorrichtung 4 in den Tablettenbehälter 100 fällt.

Dieses Verfahren wird so lange wiederholt, bis die Sollanzahl 35 an Tabletten hergestellt wurde. Danach kann der Aufnahmeraum 3 geöffnet und der Tablettenbehälter 100 entnommen werden. Der Tablettenbehälter 100 ist nun mit der Sollanzahl 35 an individualisierten Tabletten mit der Sollmasse 36 des ersten Wirkstoffs 14 gefüllt.

In Fig. 4b ist eine schematisch vereinfachte Querschnittsansicht einer beispielshaften Ausführungsform einer Wiegevorrichtung 16, einer Matrize 60 und eines Unterstempels 50 gezeigt. In der gezeigten Ausführungsform ist nur eine Matrize 60 umfasst, die mit dem Unterstempel 50 eine Einheit bildet und die mittels einer Feder 65 miteinander gekoppelt sind. In Fig. 4b ist die Feder 65 zusammengedrückt, wodurch der Unterstempel 50 bis zum oberen Rand der zentralen Öffnung 60a der Matrize reicht. Dies ist beispielsweise beim Auswerfen einer gepressten Tablette sinnvoll. Die Einheit aus Unterstempel 50 und Matrize 60 ist auf der Plattform 70 angeordnet, welche mittels der Hubeinrichtung 130 in vertikaler Richtung 120 bewegbar ist. Unterhalb von der Matrize 60 und dem Unterstempel 50 ist die Wiegevorrichtung 16 angeordnet. Die Wiegevorrichtung 16 weist an ihrer

Oberseite eine Erhebung 17 auf, die formschlüssig zu einer Ausnehmung 51 an der Unterseite des Unterstempels 50 ausgebildet ist. Die Ausnehmung 51 des Unterstempels 51 liegt an einer Ausnehmung 71 in der Plattform 70 an, die zusammen einen Hohlraum bilden, der in Fig. 4b gestrichelt dargestellt ist. Durch die Ausnehmung 71 lässt sich die Wiegevorrichtung 16 durch die Plattform 70 hindurchbewegen, so dass sie mit dem Unterstempel in Kontakt kommt.

In Fig. 4c ist das System aus Fig. 4b während des Abfüllens eines Wirkstoffes gezeigt. Die Matrize 60 und der Unterstempel 50 werden durch die Federkraft der Feder 65 in vertikaler Richtung 120 auseinander gezogen, wodurch in der zentralen Öffnung 60a der Matrize 60 eine Mulde entsteht, in die der Wirkstoff gefüllt wird. Der Unterstempel 50 liegt auf der Wiegevorrichtung 16 auf, so dass die Erhebung 17 formschlüssig in die Aufnahme 51 eingreift. Die Plattform 70 ist so weit nach unten gefahren, dass der Unterstempel 50 ausschließlich auf der Wiegevorrichtung 16 und nicht auf der Plattform 70 aufliegt. Auf diese Weise misst die Wiegevorrichtung 16 die Gesamtmasse von dem Unterstempel 50, der Matrize 60 und des in die Mulde gefüllten Wirkstoffes während des Abfüllens. Der Wirkstoff wird wie üblich mittels der Dosiervorrichtung 12 eingefüllt, die in der Fig. 4b und 4c nicht dargestellt ist.

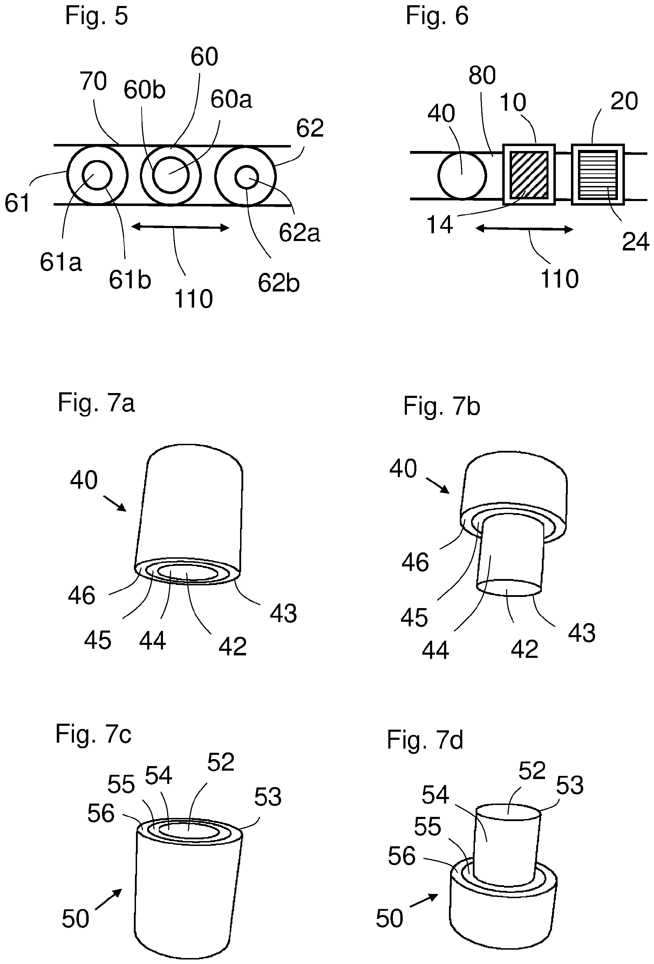

Fig. 5 zeigt eine schematisch vereinfachte Draufsicht auf eine als erste Positioniervorrichtung ausgebildete Plattform 70 gemäß einer möglichen Ausführungsform der Tablettenpresse 1. Die erste Positioniervorrichtung ist in der dargestellten Ausführungsform als Schienenvorrichtung ausgestaltet. An der ersten Positioniervorrichtung sind drei Matrizen 60, 61, 62 fixiert. Die Matrizen 60, 61, 62 weisen jeweils eine zentrale Öffnung 60a, 61a, 62a mit unterschiedlichen, kreisförmigen Innenumfängen 60b, 61b, 62b auf. Die zentralen Öffnungen 60a, 61a, 62a reichen durch die Plattform 70 hindurch, so dass die Stempel 40, 50 durch die Plattform 70 nicht blockiert werden. Durch Translation der ersten Positioniervorrichtung in horizontaler Richtung 110 lässt sich einstellen, welche der Matrizen 60, 61, 62 oberhalb des Unterstempels 50 angeordnet wird. Auf diese Weise lässt sich ein Sollumfang der hergestellten Tabletten einstellen, so dass Tabletten mit einer großen Bandbreite von Sollmassen 36 des ersten Wirkstoffs 14 mittels der Tablettenpresse 1 hergestellt werden können.

Alternativ sind statt der kreisförmigen Form der zentralen Öffnungen 60a, 61a, 62a ovale oder andere Formen vorgesehen, wodurch sich die Form der Tabletten anpassen lässt.

Fig. 6 zeigt eine schematisch vereinfachte Draufsicht auf die zweite Positioniervorrichtung 80. Diese ist in der dargestellten Ausführungsform als Schienenvorrichtung ausgeführt. An der Schienenvorrichtung ist der Oberstempel 40, der erste Materialbehälter 10 und, in der gezeigten Ausführungsform, ein zweiter Materialbehälter 20 fixiert, die sich mittels der Schienenvorrichtung durch Translation in horizontaler Richtung 110 wechselweise oberhalb des Unterstempels 50 anordnen lassen.

Der zweite Materialbehälter 20 ist mit einem zweiten Wirkstoff 24 zur Herstellung einer Zweischichttablette gefüllt. Dazu wird z.B. nach dem Pressen des ersten Wirkstoffs 14 mittels der zweiten Positioniervorrichtung 80 die zweite Dosiervorrichtung des zweiten Materialbehälters 20 über dem Unterstempel 50 angeordnet und die zentrale Öffnung 60a der auf diesem angeordneten Matrize 60 mit dem zweiten Wirkstoff 24 befüllt. Anschließend wird der Oberstempel 40 über den Unterstempel 50 bewegt und die Zweischichttablette gepresst. Zum Herstellen einer Zweischichttablette mit nebeneinanderliegenden Schichten wird eine erste Ecke der Matrize 60 mittels der ersten Dosiervorrichtung 12 mit dem ersten Wirkstoff 14 und eine zweite Ecke der Matrize 60 mittels der zweiten Dosiervorrichtung mit dem zweiten Wirkstoff 24 befüllt und anschließend die Tablette gepresst.

Fig. 7a und 7b zeigen eine Ausführungsform eines teleskopierbaren Oberstempels 40. Der Oberstempel 40 umfasst drei teleskopierend ineinander angeordnete Oberstempelschäfte 44, 45, 46. In Fig. 7a sind alle drei Oberstempelschäfte 44, 45, 46 gemeinsam ausgelenkt, so dass der Außenumfang 43 der Druckfläche 42 des Oberstempels 40 dem Außenumfang des äußersten Oberstempelschafts 46 entspricht. In Fig. 7b ist ausschließlich der innerste Oberstempelschaft 44 ausgelenkt, so dass der Außenumfang 43 der Druckfläche 42 des Oberstempels 40 dem Außenumfang des innersten Oberstempelschafts 44 entspricht. Die Außenempfänge der Oberstempelschäfte 44, 45, 46 sind jeweils formkomplementär zu den Innenumfängen 60b, 61b, 62b der Matrizen 60, 61, 62.

Fig. 7c und 7d zeigen eine Ausführungsform eines teleskopierbaren Unterstempels 50, der spiegelbildlich zu dem Oberstempel 40 ausgebildet ist. Genau wie beim Oberstempel 40 lässt sich durch individuelle Auslenkung der Unterstempelschäfte 54, 55, 56 der Außenumfang 53 der Druckfläche 52 des Unterstempels 50 variieren.

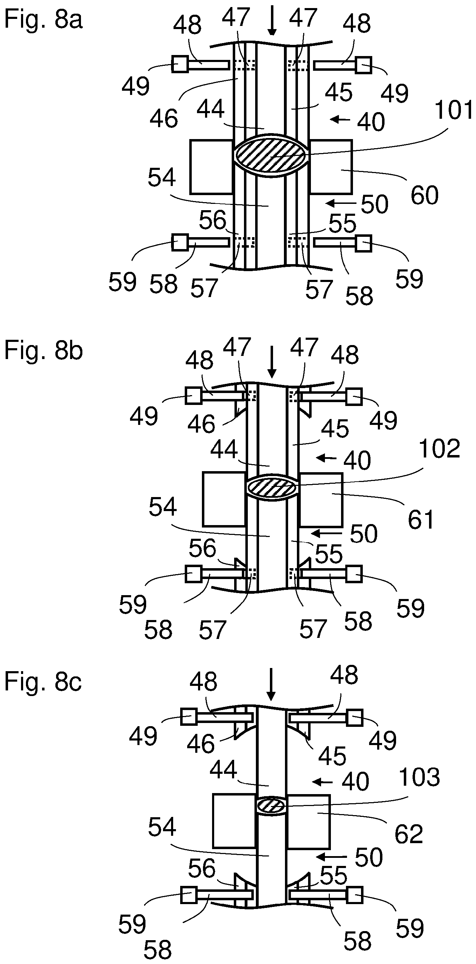

In Fig. 8a ist eine schematisch vereinfachte Querschnittsansicht eines teleskopierbaren Oberstempels 40, eines teleskopierbaren Unterstempels 50 und einer Matrize 60 während des Pressens einer großen Tablette 101 gezeigt. Dabei kommt die Matrize 60 mit der großen zentralen Öffnung 60a zum Einsatz. Zum Pressen der Tablette 101 werden sämtliche Oberstempelschäfte 44, 45, 46 und Unterstempelschäfte 54, 55, 56 genutzt. In den Oberstempelschäften 44, 45, 46 sind in der gezeigten Ausführungsform eine oder mehrere horizontale Bohrungen 47 vorgesehen, in die obere Arretierbolzen 48 mittels einer oder mehrerer schematisch angedeuteter erster Schub-und Zugvorrichtungen 49 einschiebbar sind. Analog sind in den Unterstempelschäften 54, 55, 56 in der gezeigten Ausführungsform eine oder mehrere horizontale Bohrungen 57 vorgesehen, in die untere Arretierbolzen 58 mittels einer oder mehrerer schematisch angedeuteter zweiter Schub-und Zugvorrichtungen 59 einschiebbar sind. Da die größte Matrize 60 eingesetzt wird, sind die Arretierbolzen 48, 58 so zurückgefahren, dass sie nicht in die horizontalen Bohrungen 47, 57 eingreifen. Die Schubrichtung des oberen Kolbens 96 ist durch einen Pfeil dargestellt.

Fig. 8b zeigt eine schematisch vereinfachte Querschnittsansicht während der Herstellung einer Tablette 102 mit mittlerer Größe. Dazu wird die Matrize 61 eingesetzt, die die zentrale Öffnung 61a mit mittlerer Größe aufweist. Die Arretierbolzen 48, 58 sind teilweise in die Bohrungen 47, 57 eingefahren, so dass die äußersten Stempelschäfte 46, 56 vertikal fixiert und lediglich die mittleren und die inneren Stempelschäfte 44, 45, 54, 55 ausgelenkt werden. Auf diese Weise werden die Außenumfänge 43, 53, der Druckflächen 42, 52 an den Innenumfang 61b der Matrize 61 angepasst.

Fig. 8c zeigt eine schematisch vereinfachte Querschnittsansicht während der Herstellung einer kleinen Tablette 103. Dazu wird die Matrize 62 eingesetzt, die die kleinste zentrale Öffnung 62a aufweist. Die Arretierbolzen 48, 58 sind vollständig in die Bohrungen 47, 57 eingefahren, so dass die äußersten und die mittleren Stempelschäfte 45, 46, 55, 56 vertikal fixiert und lediglich die inneren Stempelschäfte 44, 45, 54, 55 ausgelenkt werden. Auf diese Weise werden die Außenumfänge 43, 53, der Druckflächen 42, 52 an den Innenumfang 62b der Matrize 62 angepasst.

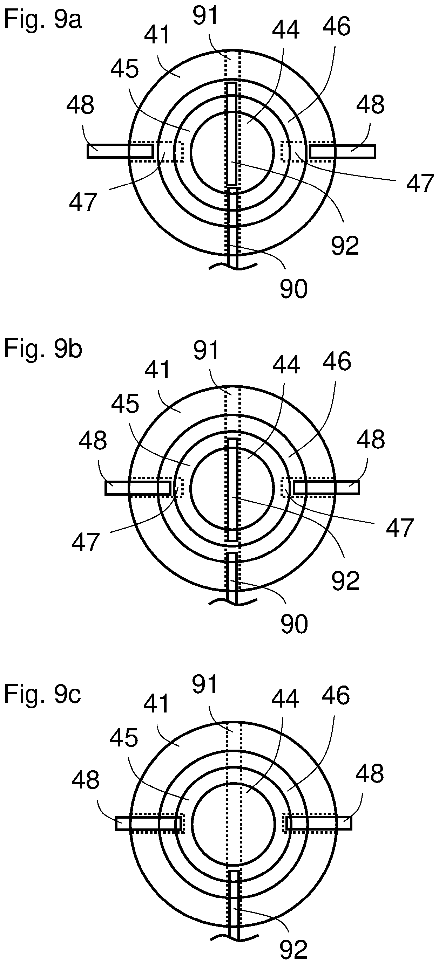

Fig. 9a zeigt eine schematisch vereinfachte Draufsicht von Fig. 8a mit zusätzlicher Darstellung des Oberstempelträgers 41 als äußerster Ring. Eine weitere horizontale Bohrung 91 ist senkrecht zu den Bohrungen 47 vorgesehen. In der Bohrung 91 wird ein Koppelbolzen 92 geführt, der mittels einer dritten Schub- und Zugvorrichtung 90 in der Bohrung 91 vor- und zurückgefahren wird.

Der obere Kolben 96 treibt in der in den Figuren 8a, 8b, 8c, 9a, 9b, 9c gezeigten Ausführungsform ausschließlich den inneren Oberstempelschaft 44 direkt an, während ein nicht dargestellter unterer Kolben ausschließlich den inneren Unterstempelschaft 54 direkt antreibt. Mittels des Koppelbolzens 92 werden die Oberstempelschäfte 44, 45, 46 gegebenenfalls miteinander gekoppelt, so dass sie synchron auf und ab bewegt werden. Für den Unterstempel 50 ist dies analog vorgesehen. In Fig. 9a koppelt der Koppelbolzen 92 alle drei Oberstempelschäfte 44, 45, 46 miteinander, so dass diese zusammen vertikal ausgelenkt werden. Damit die dritte Schub- und Zugvorrichtung 90 nicht in dem Oberstempelträger 41 blockiert, ist in dem Oberstempelträger 41 eine vertikale Nut vorgesehen.

Fig. 9b zeigt eine schematisch vereinfachte Draufsicht von Fig. 8b. Der Koppelbolzen 92 koppelt lediglich den inneren Oberstempelschaft 44 mit dem mittleren Oberstempelschaft 45, während der äußere Oberstempelschaft 46 vertikal fixiert wird.

Fig. 9c zeigt eine schematisch vereinfachte Draufsicht von Fig. 8c. Der Koppelbolzen 92 ist eingefahren, so dass lediglich der innere Oberstempelschaft 44 ausgelenkt wird, während der mittlere Oberstempelschaft 45 und der äußere Oberstempelschaft 46 vertikal fixiert werden.

Im Rahmen der Erfindung sind Merkmale, die mit "insbesondere" oder "vorzugsweise" gekennzeichnet sind, als fakultative Merkmale zu verstehen.

### Bezugszeichenliste

- 1: Tablettenpresse
- 2: Pressraum
- 3: Aufnahmeraum
- 4: Transfervorrichtung
- 6: Steuerungssystem
- 10: erster Materialbehälter
- 12: erste Dosiervorrichtung
- 14: erster Wirkstoff
- 16: Wiegevorrichtung
- 17: Erhebung
- 20: zweiter Materialbehälter
- 24: zweiter Wirkstoff
- 30: Eingabevorrichtung
- 31: Datenanzeige
- 32, 33, 34: Eingabemittel
- 35: Sollanzahl
- 36: Sollmasse
- 37: Zweitschichtsollmasse
- 40: Oberstempel
- 41: Oberstempelträger
- 42: Druckfläche
- 43: Außenumfang
- 44, 45, 46: Oberstempelschäfte
- 47: horizontale Bohrung
- 48: oberer Arretierbolzen
- 49: erste Schub- und Zugvorrichtung
- 50: Unterstempel
- 51: Ausnehmung
- 52: Druckfläche
- 53: Außenumfang
- 54, 55, 56: Unterstempelschäfte
- 57: horizontale Bohrung
- 58: unterer Arretierbolzen
- 59: zweite Schub- und Zugvorrichtung
- 60, 61, 62: Matrize
- 60a, 61a, 62a: zentrale Öffnung
- 60b, 61b, 62b: Innenumfang
- 65: Feder
- 70: Plattform
- 71: Ausnehmung
- 80: zweite Positioniervorrichtung
- 90: dritte Schub- und Zugvorrichtung
- 91: horizontale Bohrung
- 92: Koppelbolzen
- 94: Druckmessvorrichtung
- 96: Oberer Kolben
- 100: Tablettenbehälter
- 101, 102, 103: Tablette
- 110: Horizontale Richtung
- 120: Vertikale Richtung
- 130: Hubeinrichtung
- 131: Erste Spindel
- 132: Zweite Spindel

## Patentansprüche

1. Verfahren zum automatisierten Herstellen einer vorgebbaren Anzahl von individualisierten Tabletten (101, 102, 103) mit einer vorgebbaren Menge eines ersten Wirkstoffs (14) mittels einer Tablettenpresse (1) mit den folgenden Verfahrensschritten:
- Eingabe einer Sollanzahl (35), die die Anzahl herzustellender Tabletten (101, 102, 103) angibt, und einer Sollmasse (36), die die Menge des ersten Wirkstoffs (14) pro Tablette (101, 102, 103) angibt, in eine Eingabevorrichtung (30),
- Übermitteln der Sollanzahl (35) und der Sollmasse (36) von der Eingabevorrichtung (30) an ein Steuerungssystem (6) der Tablettenpresse (1), wobei das Steuerungssystem (6) eine erste Dosiervorrichtung (12), eine Wiegevorrichtung (16), einen auslenkbaren Oberstempel (40) und einen auslenkbaren Unterstempel (50) steuert,
- Abfüllen einer Menge des ersten Wirkstoffs (14), die der Sollmasse (36) entspricht, aus einem ersten Materialbehälter (14) mittels der ersten Dosiervorrichtung (12) in eine zentrale Öffnung (60a, 61a, 62a) einer Matrize (60, 61, 62), wobei während des Abfüllens eine Druckfläche (52) des Unterstempels (50) die zentrale Öffnung (60a, 61a, 62a) auf einer Seite verschließt und mittels der Wiegevorrichtung (16), auf die der Unterstempel (50) und die Matrize (60, 61, 62) während des Abfüllens abgesetzt werden, die in die zentrale Öffnung (60a, 61a, 62a) abgefüllte Menge des ersten Wirkstoffs (14) gemessen wird,
- Pressen des ersten Wirkstoffs (14) durch Auslenken einer Druckfläche (42) des Oberstempels (40) in die zentrale Öffnung (60a, 61a, 62a),
- Auslösen einer gepressten Tablette (101, 102, 103) aus der Matrize (60, 61, 62) durch Auslenken der Druckfläche (52) des Unterstempels (52) in die zentrale Öffnung (60a, 61a, 62a),
- Wiederholen der Verfahrensschritte des Abfüllens der Menge des ersten Wirkstoffs (14), des Pressens des ersten Wirkstoffs (14) und des Auslösens der gepressten Tablette (101, 102, 103), bis die Anzahl der gepressten Tabletten (101, 102, 103) der Sollanzahl (35) entspricht.

2. Verfahren nach Anspruch 1, **dadurch gekennzeichnet, dass** der Außenumfang (43) der Druckfläche (42) des Oberstempels (40) zum Pressen des ersten Wirkstoffs (14) an den Innenumfang (60b, 61b, 62b) der Matrize (60, 61, 62) angepasst wird, indem eine Anzahl von vertikal teleskopierend ineinander angeordneten Oberstempelschäften (44, 45, 46) des Oberstempels (40) nach unten ausgelenkt werden, wobei der Außenumfang (43) des äußeren ausgelenkten Oberstempelschafts (44, 45, 46) dem Innenumfang (60b, 61b, 62b) der Matrize (60, 61, 62) entspricht.

3. Verfahren nach Anspruch 1 oder 2, **dadurch gekennzeichnet, dass** der Außenumfang (53) der Druckfläche (52) des Unterstempels (50) vor dem Abfüllen der Menge des ersten Wirkstoffs (14) an den Innenumfang (60b, 61b, 62b) der Matrize (60, 61, 62) angepasst wird, indem eine Anzahl von vertikal teleskopierend ineinander angeordneten Unterstempelschäften (54, 55, 56) des Unterstempels (50) nach oben ausgelenkt werden, wobei der Außenumfang (53) des äußeren ausgelenkten Unterstempelschafts (54, 55, 56) dem Innenumfang (60b, 61b, 62b) der Matrize (60, 61, 62) entspricht.

4. Verfahren nach einem der Ansprüche 1 bis 3, **dadurch gekennzeichnet, dass** die Masse des in die zentrale Öffnung (60a, 61a, 62a) abgefüllten ersten Wirkstoffes (14) gemessen wird, indem vor dem Abfüllen die Matrize (60, 61, 62) und der Unterstempel (50) auf der Wiegevorrichtung (16) abgesetzt werden, insbesondere mittels wenigstens einer Hubeinrichtung (130), wobei die Wiegevorrichtung (16) während des Abfüllens die auf sie wirkende Gesamtmasse misst, die die Masse der Matrize (60, 61, 62), die Masse des Unterstempels (50) sowie die Masse des in die zentrale Öffnung (60a, 61a, 62a) gefüllten ersten Wirkstoffs (14) umfasst, wobei die Matrize (60, 61, 62) und der Unterstempel (50) nach dem Abfüllen und vor dem Pressen des ersten Wirkstoffes (14) angehoben werden, insbesondere mittels der wenigstens einen Hubeinrichtung (130).

5. Verfahren nach einem der Ansprüche 1 bis 4, **dadurch gekennzeichnet, dass** der auf Unterstempel (50) und/oder den Oberstempel (40) wirkende Druck an der wenigstens einen Hubeinrichtung (130), insbesondere mittels einer Druckmesseinrichtung, gemessen wird.

6. Verfahren nach einem der Ansprüche 1 bis 5, **dadurch gekennzeichnet, dass** vor dem Abfüllen der Menge des ersten Wirkstoffs (14) die erste Dosiervorrichtung (12) mittels einer zweiten Positioniervorrichtung (80), insbesondere einer in horizontaler Richtung (110) bewegbaren Schienenvorrichtung, vertikal über der zentralen Öffnung (60a, 61a, 62a) angeordnet wird, wobei nach dem Abfüllen der Menge des ersten Wirkstoffs (14) und vor dem Pressen des ersten Wirkstoffs (14) der Oberstempel (40) mittels der zweiten Positioniervorrichtung (80) vertikal über der zentralen Öffnung (60a, 61a, 62a) angeordnet wird, wobei die erste Dosiervorrichtung (12) insbesondere auch in vertikaler Richtung bewegbar an der zweiten Positioniervorrichtung (80) angeordnet, insbesondere fixiert, ist.

7. Verfahren zum Herstellen einer vorgebbaren Anzahl individualisierter Zweischichttabletten mit einer vorgebbaren Menge des ersten Wirkstoffs (14) und einer vorgebbaren Menge eines zweiten Wirkstoffs (24), wobei die Verfahrensschritte nach Anspruch 6 mit folgenden zusätzlichen Verfahrensschritten ausgeführt werden:
- Eingeben einer Zweitschichtsollmasse (37) in die Eingabevorrichtung (30) und Übermitteln der Zweitschichtsollmasse (37) an das Steuerungssystem (6), wobei die Zweitschichtsollmasse (37) die Menge des zweiten Wirkstoffs (24) pro Zweischichttablette angibt,
- Positionieren einer zweiten Dosiervorrichtung eines zweiten Materialbehälters (20) mittels der zweiten Positioniervorrichtung (80) über der zentralen Öffnung (60a, 61a, 62a),
- Abfüllen einer Menge eines zweiten Wirkstoffs (24), die der Zweitschichtsollmasse (37) entspricht, mittels der zweiten Dosiervorrichtung in die zentrale Öffnung (60a, 61a, 62a),
- Positionieren des Oberstempels (40) mittels der zweiten Positioniervorrichtung (80) über der zentralen Öffnung (60a, 61a, 62a),
- Pressen des zweiten Wirkstoffs (14) durch Auslenken der Druckfläche (42) des Oberstempels (40) in die zentrale Öffnung (60a, 61a, 62a),
wobei bis auf das Eingeben der Zweitschichtsollmasse (37) und das Übermitteln der Zweitschichtsollmasse (37) alle zusätzlichen Verfahrensschritte nach dem Pressen des ersten Wirkstoffs (14) und vor dem Auslösen der gepressten Tablette (101, 102, 103) ausgeführt werden.

8. Tablettenpresse (1) zur automatisierten Herstellung einer vorgebbaren Anzahl von individualisierten Tabletten (101, 102, 103) mit einer vorgebbaren Menge eines ersten Wirkstoffs (14), umfassend einen Pressraum (2), einen mit dem ersten Wirkstoff (14) befüllbaren ersten Materialbehälter (10), eine erste Dosiervorrichtung (12), ein Steuerungssystem (6) und eine Eingabevorrichtung (30), wobei der Pressraum (2) einen auslenkbaren Oberstempel (40), einen auslenkbaren Unterstempel (50), wenigstens eine Matrize (60, 61, 62) und eine unter der wenigstens einen Matrize (60, 61, 62) angeordnete Wiegevorrichtung (16) aufweist, mittels der eine Masse eines in eine zentrale Öffnung (60a, 61a, 62a) der Matrize (60, 61, 62) gefüllten Materials durch Absetzen der Matrize (60, 61, 62) und des Unterstempels (50) auf der Wiegevorrichtung (16) messbar ist, wobei die Eingabevorrichtung (30) dazu eingerichtet ist, eine Sollanzahl (35) und eine Sollmasse (36) der herzustellenden individualisierten Tabletten (101, 102, 103) an das Steuerungssystem (6) zu übermitteln, und wobei das Steuerungssystem (6) dazu eingerichtet ist, Messdaten der Wiegevorrichtung (16) auszulesen und die erste Dosiervorrichtung (12), den Oberstempel (40) und den Unterstempel (50) derart anzusteuern, dass eine Menge des ersten Wirkstoffs (14), die der Sollmasse (36) entspricht, in die zentrale Öffnung (60a, 61a, 62a) der Matrize (60, 61, 62) gefüllt und mittels des Oberstempels (40) und des Unterstempels (50) zu einer Tablette (101, 102, 103) gepresst wird, und diesen Vorgang so oft zu wiederholen, bis die Sollanzahl (35) an Tabletten (101, 102, 103) hergestellt wurde.

9. Tablettenpresse (1) nach Anspruch 8, **dadurch gekennzeichnet, dass** der Oberstempel (40) wenigstens zwei vertikal teleskopierend ineinander angeordnete Oberstempelschäfte (44, 45, 46) umfasst, mittels derer ein Außenumfang (43) einer Druckfläche (42) des Oberstempels (40) durch individuelle Auslenkung der Oberstempelschäfte (44, 45, 46) an den Innenumfang (60b, 61b, 62b) der zentralen Öffnung (60a, 61a, 62a) der wenigstens einen Matrize (60, 61, 62) anpassbar ist.

10. Tablettenpresse (1) nach Anspruch 8 oder 9, **dadurch gekennzeichnet, dass** der Unterstempel (50) wenigstens zwei vertikal teleskopierend ineinander angeordnete Unterstempelschäfte (54, 55, 56) umfasst, mittels derer ein Außenumfang (53) einer Druckfläche (52) des Unterstempels (50) durch individuelle Auslenkung der Unterstempelschäfte (54, 55, 56) an den Innenumfang (60b, 61b, 62b) der zentralen Öffnung (60a, 61a, 62a) der wenigstens einen Matrize (60, 61, 62) anpassbar ist.

11. Tablettenpresse (1) nach Anspruch 9 oder 10, **dadurch gekennzeichnet, dass** der Pressraum (2) eine erste Schub- und Zugvorrichtung (49) umfasst, mittels derer wenigstens ein oberer Arretierbolzen (48) in eine horizontale Bohrung (47) in Seitenwände der Oberstempelschäfte (44, 45, 46) einschiebbar ist, um eine festlegbare Anzahl der Oberstempelschäfte (44, 45, 46) vertikal zu fixieren, wobei insbesondere der Pressraum (2) eine zweite Schub- und Zugvorrichtung (59) umfasst, mittels derer wenigstens ein unterer Arretierbolzen (58) in eine horizontale Bohrung (57) in Seitenwände der Unterstempelschäfte (54, 55, 56) einschiebbar ist, um eine festlegbare Anzahl der Unterstempelschäfte (54, 55, 56) vertikal zu fixieren.

12. Tablettenpresse (1) nach einem der Ansprüche 8 bis 11, **dadurch gekennzeichnet, dass** der die Tablettenpresse (1) wenigstens eine Hubeinrichtung (130) umfasst, die dazu eingerichtet ist, die wenigstens eine Matrize (60, 61, 62) und den Unterstempel (50) anzuheben und abzusenken, insbesondere in vertikaler Richtung (120).

13. Tablettenpresse (1) nach einem der Ansprüche 8 bis 12, **dadurch gekennzeichnet, dass** eine zweite Positioniervorrichtung (80) umfasst ist, mittels derer wechselweise der Oberstempel (40) und die erste Dosiervorrichtung (12), und insbesondere eine zweite Dosiervorrichtung eines zweiten Materialbehälters (20), vertikal über der zentralen Öffnung (60a, 61a, 62a) der über dem Unterstempel (50) angeordneten Matrize (60, 61, 62) anordbar sind.

14. Tablettenpresse (1) nach Anspruch 13, **dadurch gekennzeichnet, dass** die zweite Positioniervorrichtung (80) eine Schienenvorrichtung ist, an der die erste Dosiervorrichtung (12) und der Oberstempel (40) angeordnet, insbesondere fixiert, und in horizontaler Richtung (110) bewegbar sind, wobei die erste Dosiervorrichtung (12) insbesondere auch in vertikaler Richtung (120) bewegbar an der Schienenvorrichtung angeordnet, insbesondere fixiert, ist.

15. Tablettenpresse (1) nach einem der Ansprüche 8 bis 14, **dadurch gekennzeichnet, dass** der Unterstempel (50) an seiner Unterseite eine Ausnehmung (51) aufweist, die formschlüssig zu einer Erhebung (17) an der Oberseite der Wiegevorrichtung (16) ausgebildet ist.

## Claims

1. A method for the automatic production of a specifiable number of individualized tablets (101, 102, 103) with a specifiable quantity of a first active ingredient (14) by means of a tablet press (1), having the following method steps:
- inputting a nominal number (35) which indicates the number of tablets (101, 102, 103) to be produced, and a nominal mass (36) which indicates the quantity of the first active ingredient (14) per tablet (101, 102, 103), into an input device (30),
- transmitting the nominal number (35) and the nominal mass (36) from the input device (30) to a control system (6) of the tablet press (1), wherein the control system (6) controls a first dosing device (12), a weighing device (16), an extendable upper punch (40) and an extendable lower punch (50),
- filling a quantity of the first active ingredient (14), which corresponds to the nominal mass (36), from a first material container (14) into a central opening (60a, 61a, 62a) of a die (60, 61, 62) by means of the first dosing device (12), wherein, during filling, a pressure face (52) of the lower punch (50) closes the central opening (60a, 61a, 62a) on one side, and the quantity of the first active ingredient (14) filled into the central opening (60a, 61a, 62a) is measured by means of the weighing device (16) on which the lower punch (50) and the die (60, 61, 62) are placed during filling,
- pressing the first active ingredient (14) by extending a pressure face (42) of the upper punch (40) into the central opening (60a, 61a, 62a),
- releasing a pressed tablet (101, 102, 103) from the die (60, 61, 62) by extending the pressure face (52) of the lower punch (52) into the central opening (60a, 61a, 62a),
- repeating the method steps of filling the quantity of the first active ingredient (14), pressing the first active ingredient (14) and releasing the pressed tablet (101, 102, 103) until the number of the pressed tablets (101, 102, 103) corresponds to the nominal number (35).

2. The method according to Claim 1, **characterized in that** the outer circumference (43) of the pressure face (42) of the upper punch (40) for pressing the first active ingredient (14) is adapted to the inner circumference (60b, 61b, 62b) of the die (60, 61, 62) **in that** a number of vertically telescoping upper punch shafts (44, 45, 46) of the upper punch (40) arranged inside each other are extended downwards, wherein the outer circumference (43) of the outer extended upper punch shaft (44, 45, 46) corresponds to the inner circumference (60b, 61b, 62b) of the die (60, 61, 62).

3. The method according to Claim 1 or 2, **characterized in that**, prior to filling the quantity of the first active ingredient (14), the outer circumference (53) of the pressure face (52) of the lower punch (50) is adapted to the inner circumference (60b, 61b, 62b) of the die (60, 61, 62) **in that** a number of vertically telescoping lower punch shafts (54, 55, 56) of the lower punch (50) arranged inside each other are extended upwards, wherein the outer circumference (53) of the outer extended lower punch shaft (54, 55, 56) corresponds to the inner circumference (60b, 61b, 62b) of the die (60, 61, 62).

4. The method according to any one of Claims 1 to 3, **characterized in that** the mass of the first active ingredient (14) filled into the central opening (60a, 61a, 62a) is measured **in that**, prior to filling, the die (60, 61, 62) and the lower punch (50) are placed on the weighing device (16), in particular by means of at least one lifting device (130), wherein, during filling, the weighing device (16) measures the total mass acting on it, which comprises the mass of the die (60, 61, 62), the mass of the lower punch (50) as well as the mass of the first active ingredient (14) filled into the central opening (60a, 61a, 62a), wherein the die (60, 61, 62) and the lower punch (50) are lifted after filling and prior to pressing the first active ingredient (14), in particular by means of the at least one lifting device (130).

5. The method according to any one of Claims 1 to 4, **characterized in that** the pressure acting on the lower punch (50) and/or the upper punch (40) is measured at the at least one lifting device (130), in particular by means of a pressure measuring device.

6. The method according to any one of Claims 1 to 5, **characterized in that**, prior to filling the quantity of the first active ingredient (14), the first dosing device (12) is arranged vertically above the central opening (60a, 61a, 62a) by means of a second positioning device (80), in particular a rail device that can be moved in a horizontal direction (110), wherein the upper punch (40) is arranged vertically above the central opening (60a, 61a, 62a) by means of the second positioning device (80) after filling the quantity of the first active ingredient (14) and prior to pressing the first active ingredient (14), wherein the first dosing device (12) is arranged, in particular fixed, on the second positioning device (80) so as to be movable in particular in a vertical direction as well.

7. A method for the production of a specifiable number of individualized two-layer tablets with a specifiable quantity of the first active ingredient (14) and a specifiable quantity of a second active ingredient (24), wherein the method steps according to Claim 6 are executed with the following additional method steps:
- inputting a second-layer nominal mass (37) into the input device (30) and transmitting the second-layer nominal mass (37) to the control system (6), wherein the second-layer nominal mass (37) indicates the quantity of the second active ingredient (24) per two-layer tablet,
- positioning a second dosing device of a second material container (20) above the central opening (60a, 61a, 62a) by means of the second positioning device (80),
- filling a quantity of a second active ingredient (24), which corresponds to the second-layer nominal mass (37), into the central opening (60a, 61a, 62a) by means of the second dosing device,
- positioning the upper punch (40) above the central opening (60a, 61a, 62a) by means of the second positioning device (80),
- pressing the second active ingredient (14) by extending the pressure face (42) of the upper punch (40) into the central opening (60a, 61a, 62a),
wherein, except for inputting the second-layer nominal mass (37) and transmitting the second-layer nominal mass (37), all of the additional method steps are executed after pressing the first active ingredient (14) and prior to releasing the pressed tablet (101, 102, 103).

8. A tablet press (1) for the automatic production of a specifiable number of individualized tablets (101, 102, 103) with a specifiable quantity of a first active ingredient (14), comprising a press chamber (2), a first material container (10) which can be filled with the first active ingredient (14), a first dosing device (12), a control system (6) and an input device (30), wherein the press chamber (2) has an extendable upper punch (40), an extendable lower punch (50), at least one die (60, 61, 62) and a weighing device (16) arranged under the at least one die (60, 61, 62), by means of which a mass of a material filled into a central opening (60a, 61a, 62a) of the die (60, 61, 62) can be measured by placing the die (60, 61, 62) and the lower punch (50) on the weighing device (16), wherein the input device (30) is designed to transmit a nominal number (35) and a nominal mass (36) of the individualized tablets (101, 102, 103) to be produced to the control system (6), and wherein the control system (6) is designed to read out measured data from the weighing device (16) and to actuate the first dosing device (12), the upper punch (40) and the lower punch (50) in such a way that a quantity of the first active ingredient (14), which corresponds to the nominal mass (36), is filled into the central opening (60a, 61a, 62a) of the die (60, 61, 62) and is pressed by means of the upper punch (40) and the lower punch (50) into a tablet (101, 102, 103), and to repeat said process until such time as the nominal number (35) of tablets (101, 102, 103) has been produced.

9. The tablet press (1) according to Claim 8, **characterized in that** the upper punch (40) comprises at least two vertically telescoping upper punch shafts (44, 45, 46) arranged inside each other, by means of which an outer circumference (43) of a pressure face (42) of the upper punch (40) can be adapted to the inner circumference (60b, 61b, 62b) of the central opening (60a, 61a, 62a) of the at least one die (60, 61, 62) by individually extending the upper punch shafts (44, 45, 46).

10. The tablet press (1) according to Claim 8 or 9, **characterized in that** the lower punch (50) comprises at least two vertically telescoping lower punch shafts (54, 55, 56) arranged inside each other, by means of which an outer circumference (53) of a pressure face (52) of the lower punch (50) can be adapted to the inner circumference (60b, 61b, 62b) of the central opening (60a, 61a, 62a) of the at least one die (60, 61, 62) by individually extending the lower punch shafts (54, 55, 56).

11. The tablet press (1) according to Claim 9 or 10, **characterized in that** the press chamber (2) comprises a first pushing and pulling device (49), by means of which at least one upper locking bolt (48) can be pushed into a horizontal hole (47) in side walls of the upper punch shafts (44, 45, 46) in order to vertically fix a definable number of the upper punch shafts (44, 45, 46), wherein the press chamber (2) in particular comprises a second pushing and pulling device (59), by means of which at least one lower locking bolt (58) can be pushed into a horizontal hole (57) in side walls of the lower punch shafts (54, 55, 56) in order to vertically fix a definable number of the lower punch shafts (54, 55, 56).

12. The tablet press (1) according to any one of Claims 8 to 11, **characterized in that** the tablet press (1) comprises at least one lifting device (130) which is designed to lift and to lower the at least one die (60, 61, 62) and the lower punch (50), in particular in a vertical direction (120).

13. The tablet press (1) according to any one of Claims 8 to 12, **characterized in that** a second positioning device (80) is comprised, by means of which the upper punch (40) and the first dosing device (12) and, in particular, a second dosing device of a second material container (20) can be alternately arranged vertically above the central opening (60a, 61a, 62a) of the die (60, 61, 62) arranged above the lower punch (50).

14. The tablet press (1) according to Claim 13, **characterized in that** the second positioning device (80) is a rail device on which the first dosing device (12) and the upper punch (40) are arranged, in particular fixed, and can be moved in a horizontal direction (110), wherein the first dosing device (12) is arranged, in particular fixed, on the rail device so as to be movable, including in particular in a vertical direction (120).

15. The tablet press (1) according to any one of Claims 8 to 14, **characterized in that** the lower punch (50) has a recess (51) on its lower side, which is configured with a positive fit with an elevation (17) on the upper side of the weighing device (16).

## Revendications

1. Procédé de fabrication automatisée d'un nombre prédéterminé de comprimés individualisés (101, 102, 103) avec une quantité prédéterminé d'un premier principe actif (14) au moyen d'une presse à comprimés (1), comprenant les étapes de procédé suivantes :
- saisie d'un nombre cible (35), qui indique le nombre de comprimés (101, 102, 103) à fabriquer, et d'une masse cible (36), qui indique la quantité du premier principe actif (14) par comprimé (101, 102, 103), dans un dispositif de saisie (30),
- transmission du nombre cible (35) et de la masse cible (36) du dispositif de saisie (30) à un système de commande (6) de la presse à comprimés (1), le système de commande (6) commandant un premier dispositif de dosage (12), un dispositif de pesage (16), un poinçon supérieur (40) orientable et un poinçon inférieur (50) orientable,
- remplissage d'une quantité du premier principe actif (14), qui correspond à la masse cible (36), à partir d'un premier récipient de matière (14) au moyen du premier dispositif de dosage (12) dans une ouverture centrale (60a, 61a, 62a) d'une matrice (60, 61, 62), une surface de pression (52) du poinçon inférieur (50) pénétrant pendant le remplissage dans l'ouverture centrale (60a, 61a, 62a) d'un côté, et la quantité du premier principe actif (14) versée dans l'ouverture centrale (60a, 61a, 62a) étant mesurée au moyen du dispositif de pesage (16), sur lequel le poinçon inférieur (50) et la matrice (60, 61, 62) sont déposés pendant le remplissage,
- presser le premier principe actif (14) en orientant une surface de pression (42) du poinçon supérieur (40) dans l'ouverture centrale (60a, 61a, 62a),
- libération d'un comprimé pressé (101, 102, 103) de la matrice (60, 61, 62) par orientation de la surface de pression (52) du poinçon inférieur (52) dans l'ouverture centrale (60a, 61a, 62a),
- répétition des étapes de procédé consistant à remplir la quantité du premier principe actif (14), à presser le premier principe actif (14) et à libérer le comprimé pressé (101, 102, 103) jusqu'à ce que le nombre de comprimés pressés (101, 102, 103) corresponde au nombre cible (35).

2. Procédé selon la revendication 1, **caractérisé en ce que** la circonférence extérieure (43) de la surface de pression (42) du poinçon supérieur (40) pour presser le premier principe actif (14) est adaptée à la circonférence intérieure (60b, 61b, 62b) de la matrice (60, 61, 62), en orientant vers le bas un certain nombre de tiges (44, 45, 46) de poinçon supérieur du poinçon supérieur (40), disposées verticalement de manière télescopique les unes dans les autres, la circonférence extérieure (43) de la tige de poinçon supérieur extérieure orientée (44, 45, 46) correspondant à la circonférence intérieure (60b, 61b, 62b) de la matrice (60, 61, 62).

3. Procédé selon la revendication 1 ou la revendication 2, **caractérisé en ce que** la circonférence extérieure (53) de la surface de pression (52) du poinçon inférieur (50) est adaptée à la circonférence intérieure (60b, 61b, 62b) de la matrice (60, 61, 62) avant le remplissage de la quantité du premier principe actif (14), en orientant vers le haut un certain nombre de tiges (54, 55, 56) de poinçon inférieur du poinçon inférieur (50) disposées verticalement de manière télescopique les unes dans les autres, la circonférence extérieure (53) de la tige de poinçon inférieur extérieure orientée (54, 55, 56) correspondant à la circonférence intérieure (60b, 61b, 62b) de la matrice (60, 61, 62).

4. Procédé selon l'une des revendications 1 à 3, **caractérisé en ce que** la masse du premier principe actif (14) versé dans l'ouverture centrale (60a, 61a, 62a) est mesurée en déposant, avant le remplissage, la matrice (60, 61, 62) et le poinçon inférieur (50) sur le dispositif de pesage (16), notamment au moyen d'au moins un dispositif de levage (130), le dispositif de pesage (16) mesurant la masse totale qui lui est appliquée pendant le remplissage, qui comprend la masse de la matrice (60, 61, 62), la masse du poinçon inférieur (50) ainsi que la masse du premier principe actif (14) versé dans l'ouverture centrale (60a, 61a, 62a), la matrice (60, 61, 62) et le poinçon inférieur (50) étant soulevés après le remplissage et avant le pressage du premier principe actif (14), notamment au moyen dudit au moins un dispositif de levage (130).

5. Procédé selon l'une des revendications 1 à 4, **caractérisé en ce que** la pression agissant sur le poinçon inférieur (50) et/ou le poinçon supérieur (40) est mesurée au niveau dudit au moins un dispositif de levage (130), en particulier au moyen d'un dispositif de mesure de pression.

6. Procédé selon l'une des revendications 1 à 5, **caractérisé en ce qu'**avant le remplissage de la quantité du premier principe actif (14), le premier dispositif de dosage (12) est disposé verticalement au-dessus de l'ouverture centrale (60a, 61a, 62a) au moyen d'un deuxième dispositif de positionnement (80), notamment un dispositif à rail mobile dans la direction horizontale (110), dans lequel, après le remplissage de la quantité du premier principe actif (14) et avant le pressage du premier principe actif (14), le poinçon supérieur (40) est disposé verticalement au-dessus de l'ouverture centrale (60a, 61a, 62a) au moyen du deuxième dispositif de positionnement (80), le premier dispositif de dosage (12) étant disposé, en particulier fixé, sur le deuxième dispositif de positionnement (80), qui est en particulier également mobile dans la direction verticale.

7. Procédé de fabrication d'un nombre prédéterminé de comprimés à deux couches individualisés avec une quantité prédéterminé du premier principe actif (14) et une quantité prédéterminé d'un deuxième principe actif (24), les étapes de procédé selon la revendication 6 étant mises en oeuvre avec les étapes de procédé supplémentaires suivantes :
- introduction d'une masse cible de deuxième couche (37) dans le dispositif de saisie (30) et transmission de la masse cible de deuxième couche (37) au système de commande (6), la masse cible de deuxième couche (37) indiquant la quantité du deuxième principe actif (24) par comprimé à deux couches,
- positionnement d'un deuxième dispositif de dosage d'un deuxième récipient de matériau (20) au moyen du deuxième dispositif de positionnement (80) au-dessus de l'ouverture centrale (60a, 61a, 62a),
- remplissage d'une quantité d'un deuxième principe actif (24) correspondant à la masse cible de deuxième couche (37) au moyen du deuxième dispositif de dosage dans l'ouverture centrale (60a, 61a, 62a),
- positionnement du poinçon supérieur (40) au-dessus de l'ouverture centrale (60a, 61a, 62a) au moyen du deuxième dispositif de positionnement (80),
- pressage du deuxième principe actif (14) en orientant la surface de pression (42) du poinçon supérieur (40) dans l'ouverture centrale (60a, 61a, 62a),
toutes les étapes supplémentaires du procédé, à l'exception de l'introduction de la masse cible de la deuxième couche (37) et de la transmission de la masse cible de la deuxième couche (37), étant mises en oeuvre après le pressage du premier principe actif (14) et avant la libération du comprimé pressé (101, 102, 103).

8. Presse à comprimés (1) pour la fabrication automatisée d'un nombre prédéterminé de comprimés individualisés (101, 102, 103) avec une quantité prédéterminé d'un premier principe actif (14), comprenant une chambre de pressage (2), un premier récipient de matériau (10), apte à être rempli avec la premier principe actif (14), un premier dispositif de dosage (12), un système de commande (6) et un dispositif de saisie (30), la chambre de pressage (2) comprenant un poinçon supérieur (40) orientable, un poinçon inférieur (50) orientable, au moins une matrice (60, 61, 62) et un dispositif de pesage (16) disposé sous ladite au moins une matrice (60, 61, 62), au moyen duquel une masse d'un matériau introduit dans une ouverture centrale (60a, 61a, 62a) de la matrice (60, 61, 62) est apte à être mesurée en déposant la matrice (60, 61, 62) et le poinçon inférieur (50) sur le dispositif de pesage (16), le dispositif de saisie (30) étant conçu pour transmettre au système de commande (6) un nombre cible (35) et une masse cible (36) des comprimés individualisés (101, 102, 103) à fabriquer, et le système de commande (6) étant conçu pour lire des données de mesure du dispositif de pesage (16) et pour commander le premier dispositif de dosage (12), le poinçon supérieur (40) et le poinçon inférieur (50) de telle sorte, qu'une quantité du premier principe actif (14), qui correspond à la masse cible (36), est introduite dans l'ouverture centrale (60a, 61a, 62a) de la matrice (60, 61, 62) et est pressée pour former un comprimé (101, 102, 103) au moyen du poinçon supérieur (40) et du poinçon inférieur (50), et ce processus étant répété autant de fois que nécessaire jusqu'à ce que le nombre cible (35) de comprimés (101, 102, 103) ait été fabriqué.

9. Presse à comprimés (1) selon la revendication 8, **caractérisée en ce que** le poinçon supérieur (40) comprend au moins deux tiges (44, 45, 46) de poinçon supérieur disposées verticalement de manière télescopique l'une dans l'autre, au moyen desquelles une circonférence extérieure (43) d'une surface de pression (42) du poinçon supérieur (40) est apte à être adaptée à la circonférence intérieure (60b, 61b, 62b) de l'ouverture centrale (60a, 61a, 62a) de ladite au moins une matrice (60, 61, 62) par une orientation individuelle des tiges (44, 45, 46) de poinçon supérieur.

10. Presse à comprimés (1) selon la revendication 8 ou la revendication 9, **caractérisée en ce que** le poinçon inférieur (50) comprend au moins deux tiges (54, 55, 56) de poinçon inférieur disposées verticalement de manière télescopique l'une dans l'autre, au moyen desquelles une circonférence extérieure (53) d'une surface de pression (52) du poinçon inférieur (50) est apte à être adaptée à la circonférence intérieure (60b, 61b, 62b) de l'ouverture centrale (60a, 61a, 62a) de ladite au moins une matrice (60, 61, 62) par orientation individuelle des tiges (54, 55, 56) de poinçon inférieur.

11. Presse à comprimés (1) selon la revendication 9 ou la revendication 10, **caractérisée en ce que** la chambre de pressage (2) comprend un premier dispositif de poussée et de traction (49), au moyen duquel au moins une broche d'arrêt supérieure (48) est apte à être insérée dans un alésage horizontal (47) dans des parois latérales des tiges (44, 45, 46) de poinçon supérieur, afin de fixer un nombre déterminable de tiges (44, 45, 46) de poinçon supérieur, la chambre de pressage (2) comprenant en particulier un deuxième dispositif de poussée et de traction (59) au moyen duquel au moins une broche d'arrêt inférieure (58) est apte à être insérée dans un alésage horizontal (57) dans des parois latérales des tiges (54, 55, 56) de poinçon inférieur, afin de fixer verticalement un nombre déterminable des tiges (54, 55, 56) de poinçon inférieur.

12. Presse à comprimés (1) selon l'une des revendications 8 à 11, **caractérisée en ce que** la presse à comprimés (1) comprend au moins un dispositif de levage (130) qui est conçu pour soulever et abaisser ladite au moins une matrice (60, 61, 62) et le poinçon inférieur (50), notamment dans la direction verticale (120).

13. Presse à comprimés (1) selon l'une des revendications 8 à 12, **caractérisée en ce qu'**elle comprend un deuxième dispositif de positionnement (80) au moyen duquel alternativement le poinçon supérieur (40) et le premier dispositif de dosage (12), et en particulier un deuxième dispositif de dosage d'un deuxième réservoir de matière (20), sont aptes à être disposés verticalement au-dessus de l'ouverture centrale (60a, 61a, 62a) de la matrice (60, 61, 62) disposée au-dessus du poinçon inférieur (50).

14. Presse à comprimés (1) selon la revendication 13, **caractérisée en ce que** le deuxième dispositif de positionnement (80) est un dispositif à rail sur lequel le premier dispositif de dosage (12) et le poinçon supérieur (40) sont agencés, en particulier fixés, et sont mobiles dans la direction horizontale (110), le premier dispositif de dosage (12) étant agencé, en particulier également fixé, de manière mobile dans la direction verticale (120) sur le dispositif à rail.

15. Presse à comprimés (1) selon l'une des revendications 8 à 14, **caractérisée en ce que** le poinçon inférieur (50) présente sur sa face inférieure un évidement (51) qui est réalisé par complémentarité de forme avec un bossage (17) sur la face supérieure du dispositif de pesage (16).
